(19) Europäisches Patentamt
European Patent Office
Office européen des brevets

(11) **EP 3 295 179 B1**

(12) **EUROPEAN PATENT SPECIFICATION**

(45) Date of publication and mention
of the grant of the patent:
**15.07.2020 Bulletin 2020/29**

(51) Int Cl.:
*G01N 33/68* (2006.01)       *H01J 49/26* (2006.01)
*H01J 49/00* (2006.01)

(21) Application number: **16792275.6**

(22) Date of filing: **10.05.2016**

(86) International application number:
**PCT/IB2016/052658**

(87) International publication number:
**WO 2016/181299 (17.11.2016 Gazette 2016/46)**

(54) **TOP DOWN PROTEIN IDENTIFICATION METHOD**

HIERARCHISCHES PROTEINIDENTIFIZIERUNGSVERFAHREN

PROCÉDÉ D'IDENTIFICATION DE PROTÉINES DESCENDANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2015 US 201562161129 P**

(43) Date of publication of application:
**21.03.2018 Bulletin 2018/12**

(73) Proprietor: **DH Technologies Development Pte.
Ltd.
Singapore 739256 (SG)**

(72) Inventor: **BABA, Takashi
Richmond Hill, Ontario L4S2C3 (CA)**

(74) Representative: **J A Kemp LLP
14 South Square
Gray's Inn
London WC1R 5JJ (GB)**

(56) References cited:
**US-A1- 2009 072 132     US-B2- 7 880 135**

• **J. LARRY CAMPBELL ET AL: "Near-Complete
Structural Characterization of
Phosphatidylcholines Using Electron Impact
Excitation of Ions from Organics", ANALYTICAL
CHEMISTRY, vol. 87, no. 11, 8 May 2015
(2015-05-08), pages 5837-5845, XP055430262, US
ISSN: 0003-2700, DOI:
10.1021/acs.analchem.5b01460**
• **TAKASHI BABA ET AL: "Electron Capture
Dissociation in a Branched Radio-Frequency Ion
Trap", ANALYTICAL CHEMISTRY, vol. 87, no. 1,
25 November 2014 (2014-11-25), pages 785-792,
XP055430279, US ISSN: 0003-2700, DOI:
10.1021/ac503773y**
• **FORNELLI ET AL.: 'Analysis of intact monoclonal
antibody IgG1 by electron transfer dissociation
Orbitrap FTMS' MOLECULAR AND CELLULAR
PROTEOMICS vol. 11, no. 12, 2012, pages 1758 -
1767, XP055329376**
• **BABA ET AL.: 'Electron capture dissociation in a
branched radio-frequency ion trap' ANALYTICAL
CHEMISTRY vol. 87, no. 1, 2014, pages 785 - 792,
XP055276199**
• **SATAKE ET AL.: 'Fast multiple electron capture
dissociation in a linear radio frequency
quadrupole ion trap' ANALYTICAL CHEMISTRY
vol. 79, no. 22, 2007, pages 8755 - 8761,
XP055246634**

## Description

### FIELD

The invention relates to mass spectrometry, and more particularly to methods and apparatus utilizing electron capture dissociation (ECD) in the mass spectrometric analysis of peptides.

### INTRODUCTION

Mass spectrometry (MS) is an analytical technique for determining the elemental composition of test substances that has both quantitative and qualitative applications. For example, MS can be used to identify unknown compounds and/or determine the structure of a particular compound by observing its fragmentation. Recently, MS has played an increasingly important role in proteomics due to the speed, specificity, and sensitivity of MS strategies in characterizing and identifying peptides and proteins.

One strategy in characterizing proteins in MS-based proteomics is a "bottom-up" approach in which protein(s) of interest are subject to enzymatic digestion (e.g., via trypsin, LysC, etc.) and one or more separations (e.g., multi-dimensional LC) prior to subjecting the peptide fragments to MS analysis ($MS^1$) or tandem MS/MS analysis ($MS^2$). In a "bottom up" $MS^2$ workflow, collision induced dissociation (CID) is typically utilized to further dissociate the precursor peptide fragments selected in the first MS stage into product ion fragments. The amino acid sequence of the precursor peptide ion can then be deduced from the masses of the product ion fragments. In CID, energetic collisions between the ionized precursors ions and inert neutral gas and/or nitrogen molecules vibrate and eventually dissociate (cleave) backbone amide bonds, thereby yielding b-type (N-terminal) and y- type (C-terminal) product ions. By identifying several of the product ion peptides, the original proteins can be determined (e.g., by referencing known sequences in a protein or genome database).

However, because CID reactions generally occur only at the weakest peptide amide bonds, incomplete fragmentation along the peptide backbone can make complete reconstruction of the peptide sequence difficult. Another key limitation to the use of CID in proteomics is the loss of post-translational modifications (PTMs) during the dissociation. PTMs (e.g., phosphorylated or sulfated functional groups), which are often only weakly bound to the peptide backbone, can be stripped from the peptide during fragmentation, thereby preventing the detection and characterization of PTMs in the $MS^2$ spectra.

As opposed to the "bottom up" approach described above, an alternative MS-based proteomics strategy utilizes a "top down" analysis in which intact proteins are subjected to dissociation in a mass spectrometer. While conventional CID generally dissociates too few sites to provide complete information to characterize the intact proteins' entire amino acid sequence, electron capture dissociation (ECD) and electron transfer dissociation (ETD) (collectively "ExD") are considered viable alternatives to CID for "top-down" sequencing of intact proteins due to ExD's more complete fragmentation of the peptide backbone. ECD, for example, utilizes ionic interactions between the precursor ion and low-energy electrons that lead to capture of the electrons by the multiply-charged precursor, which quickly induces a more extensive cleavage of the N-$\alpha$C bonds to primarily yield c-type (N-terminal) and z- type (C-terminal) product ions. ETD, on the other hand, reacts reagent ions with multiply-charged precursor ions of opposite charge to transfer electrons thereto. Because the energy of ExD-based dissociations is typically not (or less) distributed throughout the precursor peptide, weakly-bound PTMs are more likely to remain attached to the peptide for subsequent detection in the second MS analysis. One possible obstacle to "top down" approaches, however, is the complexity of the $MS^2$ spectra and the difficulty of determining the masses of multiply-charged product ions, which can vary in their charge state up to that of their multiply-charged precursor ion. Because of the ambiguity in the interpretation of "top down" $MS^2$ spectra caused by the variations in the charge states between product ions, some "top down" approaches further utilize charge state manipulation through gas phase ion-ion interactions to strip the product ions to a single charge state, e.g., via a proton transfer reaction. Moreover, while ECD has been demonstrated in "top down" analysis of known purified proteins, real-world applications involve complex, unknown samples, which can add further ambiguity in the analysis of the $MS^1$ or $MS^2$ spectra. Because these complex samples would typically be subject to on-line LC separation to eliminate interference from other analytes, accumulation time may be too short relative to the LC timescale to obtain sufficient intensity of product ions following the highly-efficient ECD.

Accordingly, there remains a need for improved methods and apparatus for mass spectrometric analysis of proteins utilizing ExD, that do not necessarily rely on multidimensional on-line separations of complex samples.

### SUMMARY

The invention is defined by the claims. In accordance with various aspects of the applicant's teachings, methods and apparatus described herein can provide for "top down" mass spectrometric analysis of proteins or peptides in a sample using ExD, in some aspects via direct infusion of the sample to the ion source without on-line LC separation, while deconvoluting the ambiguity in the ExD spectra. For example, methods and systems in accordance with various aspects of the present teachings can utilize patterns in charge-reduced species following ExD to correlate the ExD fragments with their precursor ions in order to more confidently identify the precursor ion from which the detected product ions originated. In some aspects, the methods and systems described herein can also enable the characterization of PTMs based on the deviations in the molecular weight of precursor ions that nonetheless result in identical amino acid sequences in the ExD spectra. In various aspects, the exemplary methods and systems described herein can thereby enable the ExD-MS analysis of an infused sample that contains an intact protein of interest (and in some cases other interfering analytes) so as to enable *de novo* sequencing of the complete protein, without the loss of PTM information as in CID-based proteomic strategies.

In accordance with various aspects of the applicant's teachings, a method of processing a sample is provided that comprises utilizing an ion source to generate a plurality of precursor peptide or protein ions ("precursor ions") from a sample solution containing at least one peptide (e.g., large peptides having greater than 20 amino acids and perhaps having a biological function, fragments of a digested protein) or protein (e.g., typically greater than 100 amino acids and generally exhibiting a 3D structure and biological function, for example, an intact, non-digested protein such as an antibody) and scanning a mass range of the precursor peptide or protein ions using a plurality of m/z isolation windows (e.g., a narrow sub-range of the mass range). The precursor ions within each of the m/z isolation windows can be subject to an ExD reaction, with the product ions resulting from each of the ExD reactions being detected so as to generate a plurality of ExD spectra corresponding to each of the m/z isolation windows, wherein at least a first ExD spectra corresponding to a first m/z isolation window of the m/z isolation windows exhibits one or more ExD fragment ions and one or more charge reduced species of the precursor ions within the first m/z isolation window. The method can further comprise determining a precursor charge state and a molecular weight for one or more species of the precursor ions within the first m/z isolation window at least partially based on a m/z of the said one or more species of precursor ions within the first m/z isolation window and a m/z of the one or more charge reduced species of the precursor ions. By way of non-limiting example, each of the m/z isolation windows can be a narrow m/z window (e.g., ~ 1 Da), depending on the configuration of the mass analyzer (e.g., a quadrupole mass filter) utilized to scan the mass range. Though one or more sample preparation steps (e.g., LC separation, electrophoresis, di-sulfide bond reduction, etc.) can be used prior to ionization of the sample solution to reduce the interference of other analytes within the sample on the MS analysis of the peptide(s) of interest, methods in accordance with various aspects of the present teachings can comprise introducing the sample solution to the ion source via direct infusion (e.g., without on-line LC separation).

In some aspects of the methods described herein, the first ExD spectra corresponding to the first m/z isolation window can exhibit a first charge reduced species, wherein the precursor charge state of a first species of precursor ions within the first m/z isolation window can be determined as a function of the m/z of the first charged reduced species and the m/z of the first species of precursor ions. Additionally, in some aspects, the molecular weight of the first species of precursor ions within the first m/z isolation window can be determined as a function of the determined precursor charge state and the m/z of the first species of precursor ions. In some related aspects, the first ExD spectra (i.e., the ExD spectra corresponding to the first m/z isolation window) can also exhibit a second charged reduced species, and the method can further comprise determining whether the second charge reduced species represents a multiply-reduced species of the first charged reduced species or a charged reduced species of a second species of precursor ions within the first m/z isolation window. Alternatively or additionally, when the first ExD spectra exhibits a second charged reduced species, methods in accordance with the present teachings can in some aspects determine whether the second charge reduced species represents a doubly-reduced species of the first charged reduced species or a charged reduced species of a second species of protein or peptide precursor ions within the first m/z isolation window (e.g., a peptide of a different amino acid sequence from the first species of peptide precursor ions).

In various aspects, when the first ExD spectra exhibits a second charged reduced species of a second species of precursor ions within the first m/z isolation window (e.g., a peptide of a different amino acid sequence from the first species of precursor ions), the method can also comprise determining a precursor charge state and separating fragments of the various charge states from one another for more definitive analysis. For example, in accordance with various aspects of the present teachings, the methods described herein can also utilize patterns in the ExD spectra across the mass range (e.g., patterns in the identity of the charge-reduced species resulting from the ExD reactions corresponding to different m/z isolation windows) to correlate and/or deconvolute the ExD spectra to help identify the species of precursor ion from which the detected ExD fragment ions originated. In some aspects, for example, the method can also comprise determining a precursor charge state and molecular weight for each of one or more species of precursor ions within each of the m/z isolation windows; determining a precursor charge state pattern for each of the precursor charge states of one or more species of precursor ions exhibiting a similar molecular weight across the mass range; and correlating the one or more ExD fragment ions in the ExD spectra corresponding to each respective m/z isolation window with the precursor charge state pattern for each of the one or more species of precursor ions at the respective m/z isolation window. By way of example, the precursor charge state pattern can be determined from the relative abundance of each of the one or more species of precursor ions at each of the selected m/z isolation windows. In some aspects, for example, the precursor charge state pattern can be determined from the relative intensity of the singly, charged reduced species for each of the one or more species of precursor ions in the ExD spectra corresponding to each of the selected m/z isolation windows.

In accordance with various aspects of the applicant's teachings, the method can further comprise generating an ExD spectrum for a first species selected from the one or more species of precursor ions by referring to the precursor charge state pattern, wherein differences in mass between ExD fragment ion peaks in the ExD spectra for the selected first species of the precursor ions are indicative of one or more amino acids in the selected first species of the precursor ions (so called *de novo* sequencing). In related aspects, the method can further comprise at least partially reconstructing the amino acid sequence in the selected first species of the precursor ion (e.g., based on the identity of the ExD fragment ions correlated with the selected first species of the precursor ion). Additionally in some aspects, the partially reconstructed amino acid sequence of the selected first species of the precursor ion can be compared to a database of known peptide sequences (e.g., via a homology search) to identify the peptide or protein contained within the sample.

In various exemplary aspects, the methods can further comprise comparing a plurality of the ExD fragment ion spectra corresponding to selected m/z isolation windows across the mass range, wherein similar ExD fragment ion patterns for a plurality of species of precursor ions of similar molecular weight can indicate the presence of different post-translational modifications on similar peptide amino acid sequences. That is, the small differences in molecular weight between the various species of precursor ions that exhibit similar fragmentation patterns can be assumed to differ in their PTMs (with the same or substantially the same amino acid backbone) rather than being peptides of substantially different amino acid sequences.

In accordance with various aspects of the applicant's teachings, a mass spectrometer system is provided that comprises an ion source configured to generate a plurality of precursor peptide or protein ions from a sample solution containing at least one peptide or protein, a mass analyzer (e.g., a quadrupole mass filter) configured to receive the plurality of precursor ions from the ion source, an ExD reaction cell configured to receive ions transmitted from the mass analyzer and subject the precursor ions to an ExD reaction, and a detector or mass analyzer (e.g., for mass selective detecting the precursor peptide ions and fragment ions and charged reduced species generated by the ExD reaction). The system can also comprise a controller configured to scan a mass range of the precursor ions using a plurality of m/z isolation windows to the ExD reaction cell so as to subject the precursor ions within each of the m/z isolation windows to an ExD reaction; generate a plurality of ExD spectra of product ions resulting from the ExD reactions corresponding to each of the m/z isolation windows, wherein at least a first ExD spectra corresponding to a first m/z isolation window exhibits one or more ExD fragment ions and one or more charge reduced species of the precursor ions within the first m/z isolation window; and determine a precursor charge state and a molecular weight for one or more species of the precursor ions within the first m/z isolation window at least partially based on a m/z of the said one or more species of precursor ions within the first m/z isolation window and a m/z of the one or more charge reduced species of the precursor ions (e.g., the singly, charged reduced species of the precursor peptide ion). In various aspects, the system can further comprise a pump for direct infusion of the sample solution to the ion source. In some aspects, the m/z isolation window can have a range of about 1 Da.

In some aspects, one or more of the following additional processes can be performed using the same controller described above for the data acquisition, or using another processor (i.e., computer) as an offline data processing. In accordance with various aspects of the present teachings, the first ExD spectra corresponding to the first m/z isolation window can exhibit a first charge reduced species, wherein the precursor charge state of a first species of precursor peptide or protein ions within the first m/z isolation window is determined as a function of the m/z of the first charged reduced species and the m/z of the first species of precursor peptide or protein ions, and wherein the molecular weight of the first species of precursor peptide or protein ions within the first m/z isolation window is determined as a function of the precursor charge state and the m/z of the first species of precursor peptide or protein ions. In some related aspects, the first ExD spectra can also exhibit a second charged reduced species, wherein the controller can be further configured to determine whether the second charge reduced species represents a multiply-reduced species of the first charged reduced species or a charged reduced species of a second species of precursor peptide or protein ions within the first m/z isolation window.

In some aspects, the controller can be further configured to determine a precursor charge state and molecular weight for each of one or more species of precursor ions within each of the m/z isolation windows; determine a precursor charge state pattern for each of the precursor charge states of one or more species of precursor ions exhibiting a similar molecular weight across the mass range; and correlate the one or more ExD fragment ions in the ExD spectra corresponding to each respective m/z isolation window with the precursor charge state pattern for each of the one or more species of precursor ions within the respective m/z isolation window. By way of example, the precursor charge state pattern can be determined by the controller based on the relative abundance of each of the one or more species of precursor ions within each of the m/z isolation windows. In some aspects, for example, the precursor charge state pattern can be determined by the controller based on the intensity of the singly, charged reduced species of each of the one or more species of precursor ions in the ExD spectra corresponding to each of the m/z isolation windows.

In accordance with various aspects of the present teachings, the system can be used to characterize the amino acid sequence of the peptide or protein that is present in the sample. By way of example, the controller can be configured to determine one or more amino acids in a selected first species of the precursor ions based on differences in mass between ExD fragment ion peaks in the ExD spectra corresponding to the m/z isolation window of the first species of precursor ions. Additionally, in some aspects, the controller can be further configured to at least partially reconstruct the amino acid sequence in the selected first species of the precursor ions. In related aspects, the controller can be further configured to compare the partially reconstructed amino acid sequence of the selected first species of the precursor ions to a database of known peptide or protein sequences (e.g., a homology search) to identify the peptide or protein from the sample that was ionized to form the precursor ions.

In various aspects, the controller can be further configured to compare a plurality of the ExD fragment ion spectra corresponding to selected m/z isolation windows, wherein similar ExD fragment ion patterns for a plurality of species of precursor ions of similar molecular weight indicate the presence of different post-translational modifications on similar amino acid sequences.

These and other features of the applicant's teaching are set forth herein.

## BRIEF DESCRIPTION OF THE DRAWINGS

The foregoing and other objects and advantages of the invention will be appreciated more fully from the following further description, with reference to the accompanying drawings. The skilled person in the art will understand that the drawings, described below, are for illustration purposes only. The drawings are not intended to limit the scope of the applicant's teachings in any way.

FIG. 1, in a schematic diagram, illustrates an exemplary MS-ECD system in accordance with one aspect of various embodiments of the applicant's teachings.

FIG. 2, in schematic diagram, illustrates an exemplary method of sample analysis that can be performed with the system of FIG. 1 in accordance with one aspect of various embodiments of the applicant's teachings.

FIG. 3 depicts an exemplary MS survey scan obtained according to the exemplary method of FIGS. 1 and 2.

FIG. 4 depicts an exemplary ECD spectra for a m/z isolation window of 981 m/z obtained according to the method of FIG. 2.

FIG. 5 depicts a heat map corresponding to a plurality of ECD spectra across a plurality of m/z isolation windows.

FIG. 6 demonstratively depicts the correspondence between the ECD of FIG. 4 and the heat map of FIG. 5 and the determination of charge state and molecular weight of one or more precursor ions in accordance with various aspects of FIG. 2.

FIG. 7 demonstratively depicts the determination of charge state and molecular weight of one or more precursor ions in accordance with various aspects of FIG. 2.

FIG. 8 schematically depicts the determination of a charge state pattern and fragmentation pattern using the heat map of FIG. 5, in accordance with various aspects of FIG. 2.

FIG. 9 schematically depicts an exemplary correlation of the plurality of ECD spectra in accordance with various aspects of FIG. 2.

FIG. 10 schematically depicts the exemplary amino acid sequencing and identification of a protein based on a deconvoluted ECD spectra in accordance with various aspects of FIG. 2.

FIG. 11 schematically depicts the exemplary amino acid sequencing of a deconvoluted ECD spectra in accordance with various aspects of FIG. 2.

## DETAILED DESCRIPTION

It will be appreciated that for clarity, the following discussion will explicate various aspects of embodiments of the applicant's teachings, while omitting certain specific details wherever convenient or appropriate to do so. For example, discussion of like or analogous features in alternative embodiments may be somewhat abbreviated. Well-known ideas or concepts may also for brevity not be discussed in any great detail. The skilled person will recognize that some embodiments of the applicant's teachings may not require certain of the specifically described details in every implementation, which are set forth herein only to provide a thorough understanding of the embodiments. Similarly it will be apparent that the described embodiments may be susceptible to alteration or variation according to common general knowledge without departing from the scope of the disclosure. The following detailed description of embodiments is not to be regarded as limiting the scope of the applicant's teachings in any manner.

The term "about" and "substantially identical" as used herein, refers to variations in a numerical quantity that can occur, for example, through measuring or handling procedures in the real world; through inadvertent error in these procedures; through differences/faults in the manufacture of electrical elements; through electrical losses; as well as variations that would be recognized by one skilled in the art as being equivalent so long as such variations do not encompass known values practiced by the prior art. Typically, the term "about" means greater or lesser than the value or range of values stated by 1/10 of the stated value, e.g., $\pm 10\%$. For instance, applying a voltage of about +3V DC to an element can mean a voltage between +2.7V DC and +3.3V DC. Likewise, wherein values are said to be "substantially identical," the values may differ by up to 5%. Whether or not modified by the term "about" or "substantially" identical, quantitative values recited in the claims include equivalents to the recited values, e.g., variations in the numerical quantity of such values that can occur, but would be recognized to be equivalents by a person skilled in the art.

In accordance with various aspects of the applicant's teachings, methods and systems described herein can address ambiguities associated with the analysis of complex ExD spectra resulting from conventional "top-down" ExD-based proteomic strategies. Indeed, because of such complexity, conventional strategies of "top-down" analysis typically utilize purified samples and/or purify the samples via one or more time-consuming, complex sample preparation steps prior to mass spectrometric analysis so as to eliminate interference from other analytes within the sample. Thus, while ExD has shown promise in "top-down" analysis of known, purified samples, the technique may be inadequate in analyzing complex samples, especially when on-line LC separation is used as accumulation time of the analyte of interest within the MS system may be too short relative to the LC timescale to obtain sufficient intensity of the multitude of product ions generated by ExD. In various aspects of the present teachings, methods and systems described herein can be useful to more confidently correlate a detected ExD fragment ion and the precursor ion from which it originated by determining and utilizing patterns in the charge-reduced species generated from the precursor ions in the ExD reaction. Additionally or alternatively, methods and systems in accordance with various aspects of the present teachings can also enable the characterization of PTMs based on similarities in the ExD fragmentation profile, despite determined deviations in the molecular weight of the precursor peptides. In various aspects, the exemplary methods and systems described herein can also enable the ExD-based analysis of an infused sample (i.e., without on-line LC separation) that contains an intact protein of interest (and in some cases other interfering analytes) and/or the *de novo* sequencing of the complete protein, without loss of PTM information as in CID-based proteomic strategies.

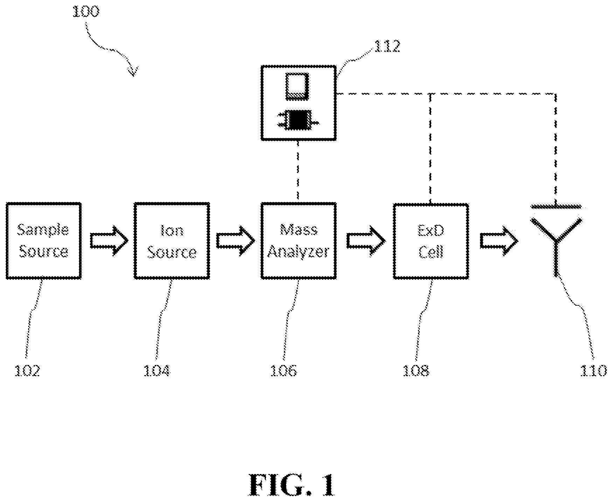

While the systems, devices, and methods described herein can be used in conjunction with many different mass spectrometer systems with fewer, more, or different components than those depicted, an exemplary mass spectrometer system 100 for use in accordance with the present teachings is illustrated schematically in FIG. 1. As shown in the exemplary embodiment depicted in FIG. 1, the mass spectrometer system 100 generally comprises a sample source 102, an ion source 104, a first mass analyzer 106, an ExD reaction cell 108, and a mass selective detector 110. As shown, the system 100 can additionally include a controller 112 operatively coupled to one or more of the first mass analyzer 106, the ExD reaction cell 108, and the detector 110 so as to control operation thereof, for example, to control the transmission of ions through and manipulation of ions within the mass analyzer 106 and ExD reaction cell 108 via the application of one or more RF/DC voltages as discussed otherwise herein and as generally known in the art and modified in accordance with the present teaching, and/or to control the manipulation/interpretation of data generated by the detector 110 in accordance with various exemplary aspects described herein. It will be appreciated that though controller 112 is depicted as a single component, one or more controllers (whether local or remote) can be configured to cause the mass spectrometer system to operate in accordance with any of the methods described herein. Indeed, the one or more controller(s) may take a hardware or software form, for example, the controller 112 may take the form of a suitably programmed computer, having a computer program stored therein that may be executed to cause the mass spectrometer to operate as otherwise described herein. Indeed, various software modules associated with the one or more controller(s) can execute programmable instructions to obtain an MS survey scan, scan precursor ions according to a m/z isolation window, subject the precursor ions to ExD reactions, receive data generated by the detector, analyze data (e.g., identify peaks), output data (including fragmentation data, ExD spectra, amino acid sequences), and perform or direct the performance of a homology search, all by way of non-limiting example.

The sample source 102 have a variety of configuration but generally is configured to contain and/or introduce a sample (e.g., a solution containing or suspected of containing a protein or peptide) to the ion source 104 for ionization thereby. As will be appreciated by a person skilled in the art, the ion source 104 can be fluidly coupled to and receive a liquid sample from a variety of liquid sample sources (e.g., through one or more conduits, channels, tubing, pipes, capillary tubes, etc.). By way of non-limiting example, the sample source 102 can comprise a reservoir of the sample to be analyzed or an input port through which the sample can be injected. In some aspects, for example, the sample source can comprise an infusion pump (e.g., a syringe pump) for continuously flowing the sample into the ion source 104. Alternatively, also by way of non-limiting example, the liquid sample to be analyzed can be in the form of an eluent from an on-line liquid chromatography column, though in preferred embodiments, one or more sample preparation steps (e.g., multi-dimensional LC separations, electrophoresis, di-sulfide bond reduction, etc.) can be performed off-line. Indeed, as otherwise discussed herein, some exemplary embodiments of the present methods and systems can enable a reduction in the sample preparation steps and/or the elimination of on-line separation techniques for some complex samples.

The ion source 104 can have a variety of configurations but is generally configured to generate ions from peptides and/ proteins within the sample of the sample source 102. In one exemplary aspect, the ion source 104 can include a conduit in direct or indirect fluid communication with the sample source 104 that terminates in an outlet end that at least partially extends into an ionization chamber. As the liquid sample is discharged from the outlet end into the ionization chamber (e.g., as a plurality of micro-droplets), peptides and/or proteins contained within the micro-droplets can be ionized (i.e., charged) by the ion source 104. As the liquid (e.g., a solvent) within the droplets evaporates, the protein or peptide ions can be released and drawn toward and through an aperture for transmission to the mass analyzer 106. It will be appreciated that a number of different devices known in the art and modified in accord with the teachings herein can be utilized as the ion source 104. By way of non-limiting example, the ion source 104 can be a electrospray ionization device, a nebulizer assisted electrospray device, a chemical ionization device, a nebulizer assisted atomization device, a photoionization device, a laser ionization device, a thermospray ionization device, and a sonic spray ionization device.

The mass analyzer 106 can have a variety of configurations but is generally configured to process (e.g., filter, scan, trap) sample ions generated by the ion source 104. By way of non-limiting example, the mass analyzer 106 can be a quadrupole rod set (e.g., Q1 in a QTRAP® Q-q-Q hybrid linear ion trap mass spectrometer, as generally described in an article entitled "Product ion scanning using a Q-q-Qlinear ion trap (Q TRAP®) mass spectrometer," authored by James W. Hager and J. C. Yves Le Blanc and published in Rapid Communications in Mass Spectrometry (2003; 17: 1056-1064). In various aspects, the mass analyzer 106 can be operated as a conventional transmission RF/DC quadrupole mass filter that can be operated to select a range of ions of interest for transmission therethrough. By way of example, the quadrupole rod set Q1 can be provided with RF/DC voltages suitable for operation in a mass-resolving mode. As will be appreciated by a person skilled in the art, taking the physical and electrical properties of Q1 into account, parameters for an applied RF and DC voltage can be selected so that Q1 establishes a quadrupolar field having an m/z passband that can be scanned across a plurality of m/z isolation windows. That is, at each m/z isolation window ions having m/z ratios falling within the passband can traverse the quadrupolar field largely unperturbed, while ions having m/z ratios falling outside the passband are degenerated by the quadrupolar field into orbital decay, and thus, be prevented from traversing the quadrupole rod set Q1. As will be appreciated by a person skilled in the art, the mass spectrometer system 100 can additionally include one or more additional elements upstream (e.g., an RF-only focusing ion guide Q0, a differential mobility filter (DMS)) or downstream (e.g., Q3) therefrom. It will also be apparent to those skilled in the art that there may be a number of ion optical elements in the system.

As shown in FIG. 1, the exemplary mass spectrometer system 100 additionally includes an ExD reaction cell 108, within which the precursor peptide or protein ions transmitted by the mass analyzer 106 can be subject to an electron capture reaction (i.e., an ETD reaction cell) or an electron transfer reaction (i.e., an ETD reaction cell), by way of non-limiting example. It will be appreciated that any ExD or ion-ion reaction device known in the art and modified in accordance with the teachings herein can be utilized as the ExD cell 108. By way of non-limiting example, the ExD cell 1 08 can be a Fourier transform mass spectrometer (FT-ICR-MS), a RF quadrupole ion trap (e.g., Q2 within a Q-q-Q triple quadrupole mass spectrometer) (see e.g., Baba et al. Electron Capture Dissociation in a Radio Frequency Ion Trap. Anal. Chem. 2004, Aug. 1; 76(15): 4263-6)and U.S. Patent Nos. 6,995,366, 7,145,139, and 7,775,034), an orbitrap-type device (see e.g., U.S. Patent No. 7,714,283), or a cross-type ion-ion reaction device (e.g., PCT Pub. No. WO2014191821).

An ECD reaction normally involves a multiply protonated molecule M interacting with a free electron to form radical species with odd number of electrons:

$$[M+nH]^{n+} + e^{-} \rightarrow [M+nH]^{(n-1)+\bullet} \rightarrow ECD\ fragments$$

In contrast to ECD, ETD instead employs reagent ions (e.g., $A^{-}$, a reagent anion) of an opposite charge to the precursor ions (e.g. radical polyaromatic anions of anthracene or fluoranthene) as electron donors in a charge transfer reaction:

$$[M+nH]^{n+} + A^{-} \rightarrow [M+nH]^{(n-1)+\bullet} \rightarrow ETD\ fragments.$$

Adding an electron to an incomplete molecular orbital of the precursor cation releases binding energy which, if sufficient to exceed a dissociation threshold, causes the fragmentation of the electron acceptor ion. In either the case of ECD or ETD, however, not all precursor ions that accept an electron necessarily dissociate. Rather, in many instances, in addition to the cleaved, fragment ions, the ExD reaction additionally produces one or more charge reduced species of the precursor ion (e.g., singly-charge reduced species $[M+nH]^{(n-1)+}$, doubly-charged reduced species $[M+nH]^{(n-2)+}$, triply-charged reduced species $[M+nH]^{(n-3)+}$, etc.).

As shown in FIG. 1, once precursor ions within each m/z isolation window are reacted within the ExD cell 108, product ions (including the charge-reduced species and fragment ions) can be transferred to one or more mass analyzers for further analysis prior to detection of the ExD product ions by the detector 110. By way of example, a mass analyzer disposed between the ExD cell 108 and the detector 110 can comprise any suitable mass spectrometer module including, but not limited to, a time of flight (TOF) mass spectrometry module, a quadrupole mass spectrometry module, a linear ion trap (LIT) module and the like, for example for scanning the product ions therefrom. In any event, ExD spectra corresponding to each of the plurality of m/z isolation windows can be acquired based on the detection of the product ions by the detector 110. Indeed, the detector 110 can have a variety of configurations, but is generally configured to detect the ions transmitted by the mass analyzer 106 and/or the ExD cell 108. By way of example, a detector (e.g., an electron multiplier, a multi-channel plate or other ion current measuring device) can be effective to detect the ions transmitted through the mass analyzer 106 and ExD cell 108. The detected ion data can be stored in memory and analyzed by a computer or computer software.

Furthermore, while not depicted, mass spectrometer system 100 can comprise any suitable number of vacuum pumps to provide a suitable vacuum or vacuum differential within and between the various elements of the mass spectrometer system 100. For example, a vacuum differential is generally applied between ion source 104 and mass analyzer 106, such that ion source 104 can be maintained at atmospheric pressure, while the mass analyzer 106 is under vacuum. While also not depicted, it will be appreciated that mass spectrometer system 100 can further comprise any suitable number of connectors, power sources, RF (radio-frequency) power sources, DC (direct current) power sources, gas sources, and any other suitable components for enabling operation of mass spectrometer system 100 in accordance with the present teachings.

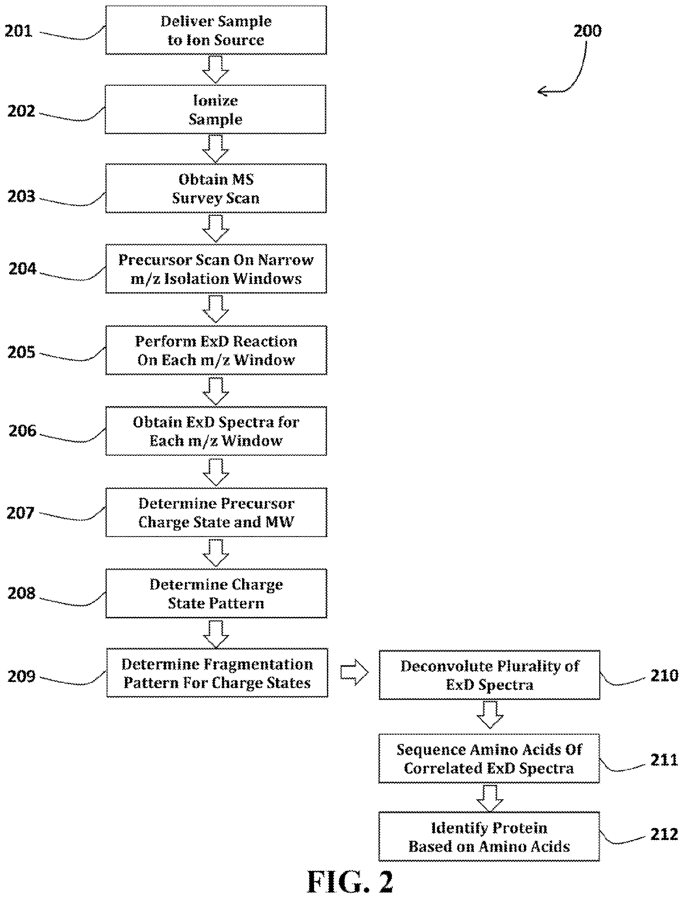

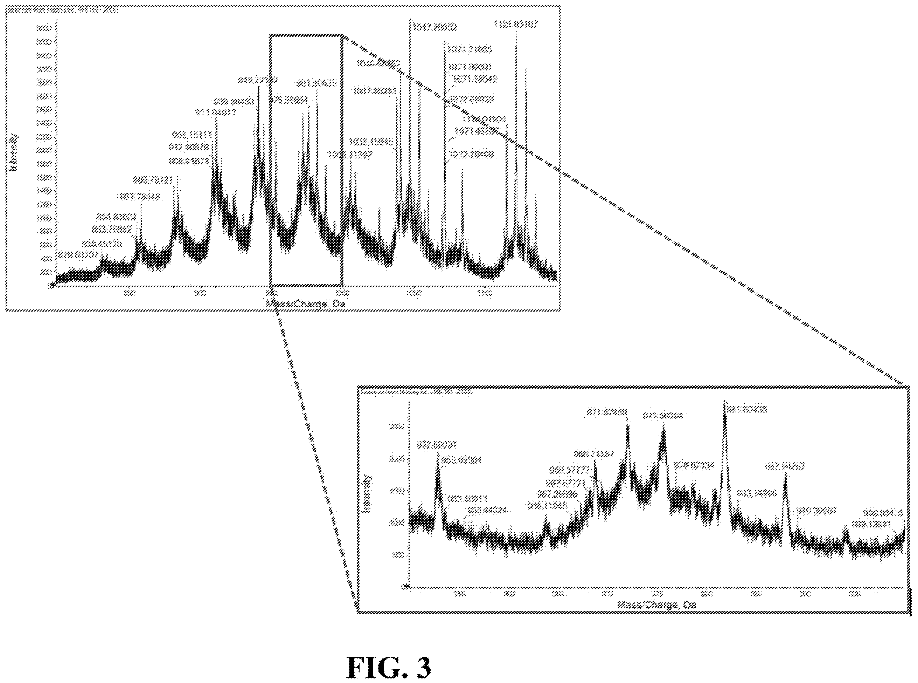

With reference now to FIG. 2, an exemplary method for operating the mass spectrometer system of FIG. 1 in accordance with various aspects of the present teachings is depicted. As shown in step 201, the method 200 can begin by delivering a sample containing a peptide or protein from a sample source 102 to the ion source 104, whereby the sample is ionized as shown in step 202. Once the peptide or proteins are ionized, in some aspects an MS survey scan can be obtained as shown in step 203. By way of example, the mass spectrometer system 100 can obtain a survey scan spectrum or a MS spectrum by operating the mass analyzer 106 in a mass filter mode in which the mass analyzer scans across a broad range of m/z, or by operating the mass analyzer 106 in non-mass selective transmission mode in the case of a TOF mass spectrometer used as the detector 110 while the ExD cell 108 is in a transparent mode (i.e., the peptide or protein ions flow through the ExD cell 108 without having an electron transferred thereto). In this manner, the detector 110 can detect every precursor ion within the broad mass range, perhaps, to identify various peaks that can be selected for further analysis in accordance with the present teachings. For example, with reference now to FIG. 3, an MS survey scan is depicted in which a sample containing non-purified carbonic anhydrase (as well as other peptides and proteins) is ionized by an electrospray ion source, then the ions were detected by a TOF mass analyzer. The range from about 950 to about 1000 Da is shown in expanded view, which in this example has been selected for further analysis. As shown in FIG. 3, each of these ranges exhibit multiple peaks, each of which can be due to the presence of multiple protein or peptide ions from different species overlapping at a given m/z.

In some aspects, a particular mass range of interest (e.g., 950-1000 Da) can be based, for example, on the MS survey scan and *a priori* knowledge of the likely m/z of a protein ion of interest, and the mass analyzer 106 can be operated so as to iteratively provide a bandpass to scan precursor ions within a plurality of m/z isolation windows across the mass range to the ExD cell 108 in step 204. The m/z isolation window can have a variety of narrow widths (e.g., less than 5 Da, about 2-5 Da, about 1 Da) and can be scanned at a variety of rates to allow for sufficient accumulation/reaction time in the ExD cell 108 in step 205. In various embodiments, for example, the mass analyzer 106 can be operated at a m/z isolation window width of about 1 Da such that only precursor peptide or protein ions with the nominally same m/z are transmitted into the ExD cell. It should be appreciated that in instances in which the sample source 102 utilizes direct infusion of the sample to the ion source 104, the quantity of precursor ions would therefore not be constrained by LC elution time (e.g., the retention time window during LC in which a particular protein or peptide would be eluted), as would be the case in front-end separation strategies that utilize on-line LC separation. As such, the accumulation/reaction time for each m/z isolation window can be selected independent of elution time, and for example, be selected such that sufficient precursor ions within the m/z isolation window can be reacted in the ExD cell 108 to provide a detectable intensity of the various product ions resulting from the ExD reaction. In various embodiments, the precursor scan of each m/z isolation window can have a transmission duration of greater than 5 minutes, by way of non-limiting example. It will further be appreciated that the step of obtaining a survey scan as in step 203, let alone selecting a sub-range of interest, may not be necessary as the system 100 could instead acquire an ExD spectrum for every precursor ion across the broad mass range. While this may eliminate any decisions about which precursor ions to select for further analysis by ExD, when the mass range of interest is large, such a process could be time-consuming and inefficient, as significant amounts of sample could be required.

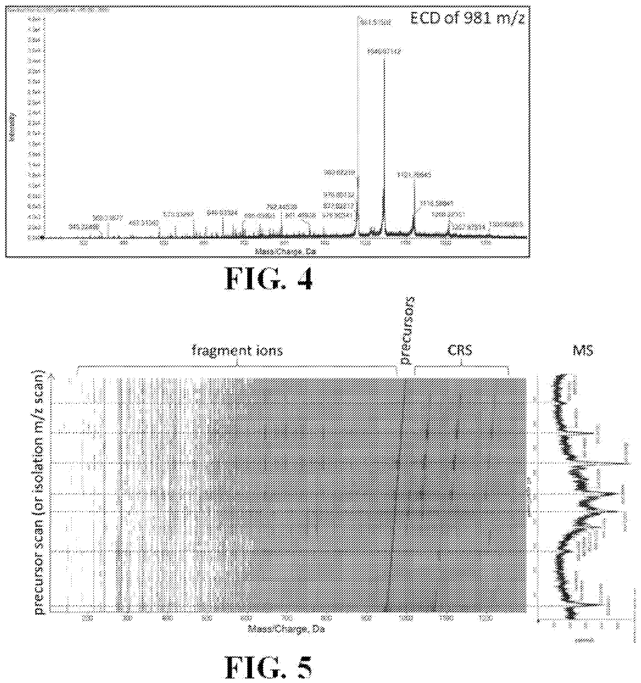

In step 205, the ions within the m/z isolation window scanned to the ExD cell 108 are then be subject to an ExD reaction (e.g., via interactions with electrons or reagent ions trapped within or transmitted through the ExD cell 108), thereby resulting in the formation of product ions, including ExD fragment ions and charge reduced species (as discussed above). In step 206, the ExD product ions (and any leftover precursor ions) can then be detected by the detector 110 so as to generate an ExD spectra for each m/z isolation window as in step 206. For example, with reference now to FIG. 4, an ECD spectra is depicted in which the mass analyzer 106 reacted precursor ions of 981 m/z (m/z isolation window width of about 1 Da) with electrons in a quadrupole-TOF mass spectrometer with ECD capability (for 6 minute spectrum accumulation), with the product ions being detected by the TOF analyzer as the detector 110. As shown in FIG. 4, the most intense peak detected following the ECD reaction is unreacted precursor ions at 981 m/z, as well as various other peaks representing product ions of both lower and higher m/z. Upon detection of the ExD reaction products for a first m/z isolation, precursor ions within the next m/z isolation window can then be scanned into the ExD reaction cell, and so on, for any number of m/z isolation windows.

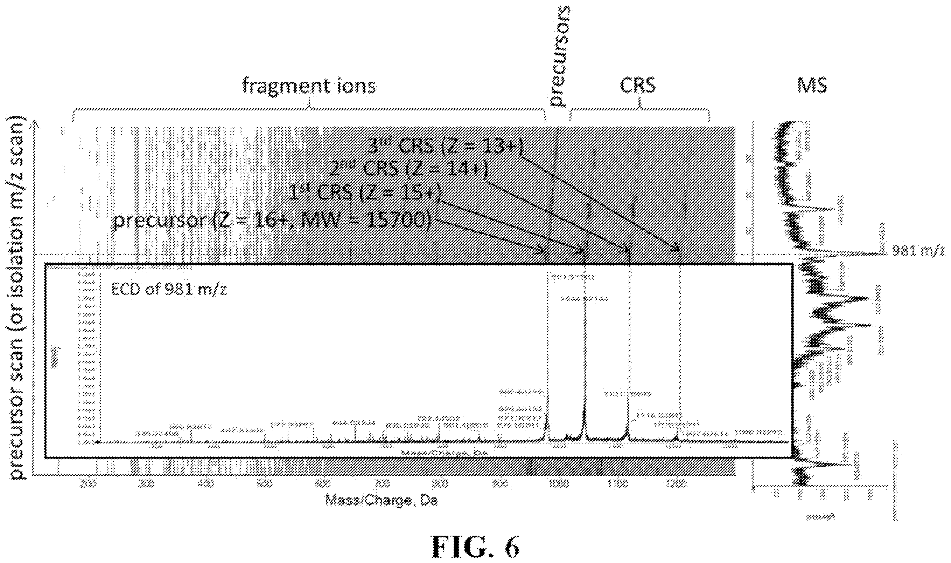

In such a manner, an ExD spectra can be obtained for each of the m/z isolation windows, which as shown in FIG. 5, can be represented by a heat map that combines a plurality of ExD spectra from the precursor scan across the exemplary mass range of interest of 950-1000 Da to indicate the intensity (darker/red = more intense) of detected product ions of m/z about 100-1300 Da generated by the plurality of ExD reactions, with each horizontal line in the heat map representing a different m/z isolation window having a width of 1 Da. Inspecting the heat map of FIG. 5 generally, it will be observed that the average intensity of product ions for particular m/z isolation windows corresponding to major peaks in the MS survey scan are generally higher. That is, as precursor ions (the intense darker/red line) are scanned across the range of m/z from 950-1000 Da in one m/z increments, the heat map indicates increased average intensity along the horizontal dotted lines that intersect the major peaks of the MS spectra (the MS spectra of FIG. 3 is flipped and rotated to demonstrate this correspondence between the precursor scan and the MS survey scan). FIG 6 further demonstrates the correspondence between the heat map of FIG. 5 and the ECD spectra for each m/z isolation window. As shown in FIG. 6 by comparing the inset of the ECD spectra of FIG. 4 with the intensity of product ions along the horizontal, dotted line, the heat map indicates the intensity of the product ions resulting from the ECD reaction of the precursor ions having an m/z of 981 Da. By way of example, the heat map of FIG. 6 at the horizontal line corresponding to precursor ions at 981 Da depicts an increased intensity of product ions at m/z of about 792 Da, about 981 Da (i.e., the precursor ion), about 1047 Da, about 1122 Da, and about 1208 Da.

Moreover, in accordance with various aspects of the present teachings, the ECD spectra can be further analyzed (e.g., by controller 112) to determine particular aspects of the product ions resulting from the ECD reaction of the precursor ions within the m/z isolation window. First, as noted above, while ExD reactions in which an electron is accepted by the precursor ion can result in fragmentation of the electron acceptor (i.e., cleavage of the protein or peptide precursor ion along the amine bonds of the peptide backbone), the energy of the ExD reaction may be insufficient to cause fragmentation such that one or more charge reduced species of the precursor ion results. That is, the addition of an electron can generate a charge reduced species (CRS) exhibiting a decreased charge relative to the peptide or protein precursor ion, without substantially changing the mass of the precursor ion (due to lack of cleavage of peptides therefrom). As such, for each precursor ion of a given m/z (i.e., $[M+nH]^{n+}$), charge reduced species (e.g., singly-charge reduced species $[M+nH]^{(n-1)+}$ and doubly-charged reduced species $[M+nH]^{(n-2)+}$) would appear in the ExD spectra at higher m/z relative to the precursor ion (i.e., same mass (m) with reduced charge (z) $\rightarrow$ higher m/z). With reference to FIG. 6, for example, it will be appreciated that the observed peaks at about 1047 Da, about 1122 Da, and about 1208 Da likely represent these charge-reduced species of the one or more precursor ions at 981 m/z, while other smaller (less intense) peaks in the ECD spectra instead represent fragment ions of both higher m/z and lower m/z relative to the precursor ions.

Indeed, in accordance with various aspects of the present teachings, in step 207 of FIG. 2 the major peaks in the ExD product ion spectra having an m/z greater than the precursor ions (i.e., the likely CRS) can be analyzed to determine the precursor charge state (i.e., the charge of the precursor ion, n in $[M+nH]^{n+}$) and/or the precursor molecular weight (e.g., in atomic mass units or amu) of the precursor ion from which the CRS was derived. By way of non-limiting example, the charge state of the 981 m/z precursor ion can be determined as a function of a difference between the m/z of the first observed CRS and the 981 m/z of the precursor ion, as follows:

$$Z_1 = \frac{Y_1}{Y_1 - X},$$

where $Z_1$ is the charge state of one species of precursor ions at 981 m/z, $Y_1$ is the m/z of the first CRS (i.e., 1046.97142 Da), and X is the m/z of the first species of precursor ions (i.e., 981.51502). Based on the above exemplary function, the precursor charge state is determined to be 16+ (i.e., n = 16 in $[M+16H]^{16+}$).

Similarly, the precursor molecular weight (i.e., the m in m/z) of the precursor ion from which the CRS was derived can be determined in step 207, for example in light of the determined precursor charge state and its 981 m/z. By way of example, the molecular weight ($M_1$) of the 981 m/z precursor ion can be determined to be about 15700 amu by the function:

$$M_1 = Z_1 \times (X - m_H),$$

where $m_H$ is the atomic mass of hydrogen.

As shown in FIG. 6, the ExD spectra at 981 m/z also exhibits additional CRS, which may represent multiply-reduced species of the same precursor ion (i.e., $Z_1 = 16+$, $M_1 \approx 15700$ amu), or alternatively, may represent a singly-charged reduced species of different precursor ion that also exhibits an m/z of 981 Da. For example, if the charge state of the ion of which the CRS at about 1122 Da is derived is confirmed to match that of the first CRS (i.e., where the precursor has $Z_1 = 16+$, the first CRS at 1046.97142 Da would exhibit a charge state of $(Z_1 - 1) = 15+$), it can be determined that the second CRS at about 1122 Da is the doubly-CRS of the precursor ion. For example, it can be determined whether the second CRS (at 1121.76645 m/z) represents a doubly-reduced species of the first CRS (at $Y_1$ = 1046.97142 Da) by the function:

$$Z_2 = \frac{Y_2}{Y_2 - Y_1},$$

where $Y_2$ is the m/z of the second charged reduced species (i.e., 1121.76645 Da). If $Z_2$ is substantially equal to ($Z_1$ - 1), then the second CRS can be assumed to represent a doubly-reduced species of the first CRS. However, if $Z_2 \neq (Z_1 - 1)$, then the second charged reduced species instead represents a charged reduced species of a second species of precursor ions within the first m/z isolation window (e.g., a peptide of a different amino acid sequence from the first species of precursor ions). Utilizing the above function, it was confirmed that the peak at about 1122 Da in the ECD spectra of 981 m/z precursor represented the 14+ doubly-reduced CRS of the same precursor ion (i.e., $Z_1 = 16+$, $M_1 \approx$ 15700 amu) as that of the first singly-reduced CRS at about 1047 Da. Likewise, using the second CRS as the base in the directly preceding function, it was also confirmed that the peak at about 1208 Da represented the 13+ triply-reduced CRS of the same precursor ion (i.e., $Z_1 = 16+$, $M_1 \approx$ 15700 amu).

It will be appreciated that the above function utilized to determine whether the second CRS represents a doubly-reduced species of the same precursor ion is one exemplary method. By way of example, when the ExD spectra exhibits a second CRS, it can alternatively be determines whether the second CRS represents a doubly-reduced species by the function:

$$M_X = (Z_1 - 1) \times (X - 2m_H).$$

If $M_X = M_1$, then the second CRS represents a doubly-reduced species of the same precursor ion. However, if $M_X \neq M_1$, then the second CRS instead represents a charged reduced species of a second species of precursor ion within the m/z isolation window (e.g., a peptide of a different amino acid sequence from the precursor ion having a $Z_1 = 16+$ and $M_1 \approx$ 15700 amu).

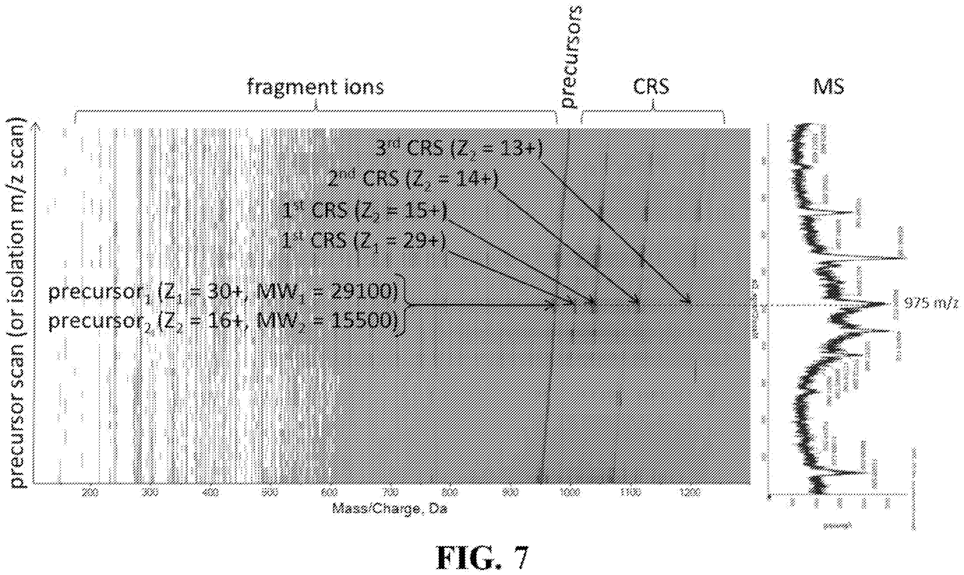

In such an exemplary manner, as shown in step 207 of FIG. 2, the charge state and molecular weight for the one or more species of precursor ions within the m/z isolation window can thus be determined (e.g., by controller 112 using a peak identification module) from the precursor and CRS peaks in the ExD spectra corresponding to each m/z isolation window. For example, with reference now to FIG. 7, the ECD spectra corresponding to an m/z isolation window at 975 Da depicts four species of CRS, each of which is analyzed to determine whether they correspond to a CRS of the same or different species of precursor ions at 975 Da. Using the exemplary relationships described above, it was determined that the precursor peak at 975 Da in the ECD spectra represented two different species of precursor ions, one (i.e., precursor$_1$) exhibiting a charge state of 30+ and a molecular weight of about 29,100 amu, and the other (i.e., precursor$_2$) exhibiting a charge state of 16+ and a molecular weight of about 15,500 amu. Specifically, the CRS peak at 1006 Da was found to represent a singly-charge reduced species of precursor$_1$, while the CRS peaks at approximately 1041 Da, 1126 Da, and 1200 Da were determined to represent singly-, doubly-, and triply-charged reduced species of precursor$_2$, respectively.

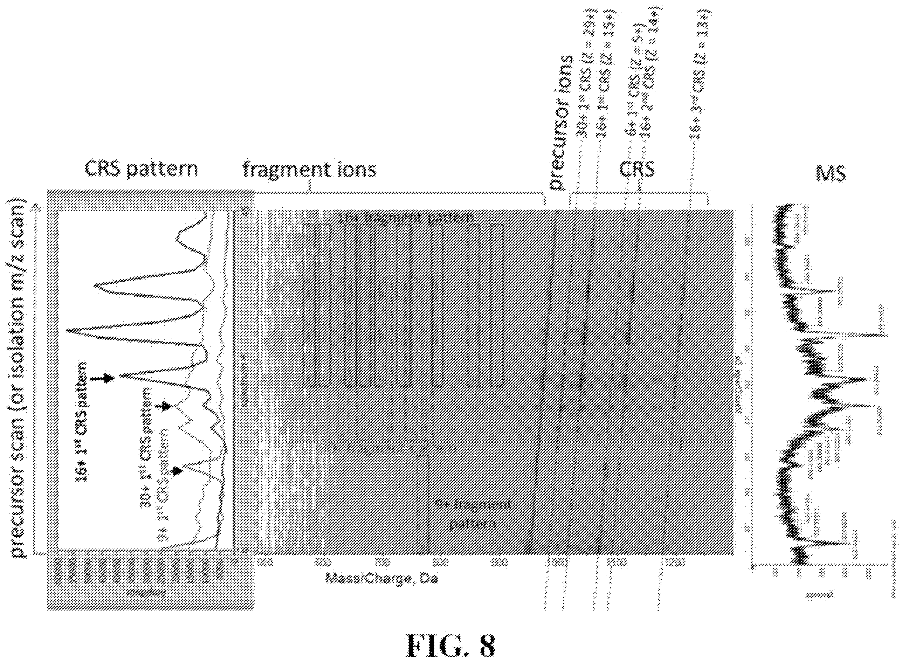

In various aspects of the present teachings, after calculating the precursor charge state and molecular weight of the precursor ions across a plurality of m/z isolation windows (in step 207), the heat map depicted in FIG. 6 can be further analyzed to determine relationships between the ExD spectra corresponding to each m/z isolation window. For example, with reference to FIG. 8, the ExD at various m/z isolation windows can be compared to determine if any patterns exist in the presence of fragment ions resulting from precursor ions having the same charge state and similar molecular weight. Indeed, methods and systems in accordance with various aspects of the present teachings utilize the presence of substantially the same fragmentation pattern across ExD spectra corresponding to different m/z isolation windows and the charge state pattern across the ExD spectra to correlate the fragment ions detected in a particular ExD spectra with the precursor ion from which the fragment ion is derived. Though differences in molecular weight of the precursor ions of the same charge state would tend to suggest that the precursors are different from one another, the present teachings attribute differences in molecular weight between the precursor ions to the presence of PTMs or small differences in the amino acid sequence so as to more confidently associate fragment ions observed in a convoluted ExD spectra with one of the several precursor ions within the m/z isolation window.

By way of example, with the assumption that small differences in molecular weight between precursor ions of similar charge state across m/z isolation windows indicate substantially similar peptide or protein precursor (albeit with minor differences in amino acid sequence or the PTMs associated therewith), a charge state pattern for the related protein precursors can be determined in step 208, based on the relative abundance of the related precursor ions across the m/z isolation windows. In the exemplary heat map of FIG. 8, for example, a charge state pattern (i.e., the CRS pattern inset) is determined for each of the 9+, 16+, 30+ charge state precursors determined in step 207 across the mass range (i.e., across a plurality of m/z isolation windows). In particular in this exemplary depicted embodiment, the charge state pattern for the precursors of the various charge states is determined based on the intensity of the singly-charged reduced species across the mass range (i.e., along the dotted line corresponding to the singly-charged reduced species of each precursor charge state). That is, the 16+ charge state pattern on the CRS pattern inset is the detected intensity at each m/z isolation window along the dotted line corresponding to 16+ 1st CRS (Z = 15+), the 30+ charge state pattern is the detected intensity along the dotted line corresponding to the 30+ 1st CRS (Z = 29+), and the 9+ charge state pattern is the detected intensity along the dotted line corresponding to the 9+ 1st CRS (Z = 8+).

In step 209, methods and systems in accordance with the present teachings can also compare the fragment ions produced from the precursor ions in different m/z isolation windows to identify at least a partial fragmentation pattern corresponding to the related precursor ions of each charge state. By way of example with specific reference to the heat map of FIG. 8, it is observed that ECD spectra of related precursor ions (i.e., similar molecular weight, same charge state) within different m/z isolation windows can exhibit similarities in the pattern of fragment ions, as demonstrated by the vertical boxes of FIG. 8. By way of example, in the 16+ fragment pattern, it can be seen that fragment ion peaks between m/z isolation windows are present at the same m/z, and moreover, vary in intensity between the m/z isolation windows in proportion to the relative intensity of the 15+ CRS of the precursors having a charge state of 16+ and a molecular weight of about 15500-16000 amu, as detected in the ECD spectra corresponding to each respective m/z isolation window. Moreover, it should be appreciated that these similar fragmentation patterns confirm that the precursor ions have substantially the same amino acid sequences (i.e., the sequences of amino acids represented by the various fragment ions are nonetheless substantially despite the differences in the calculated molecular weights between the precursors.

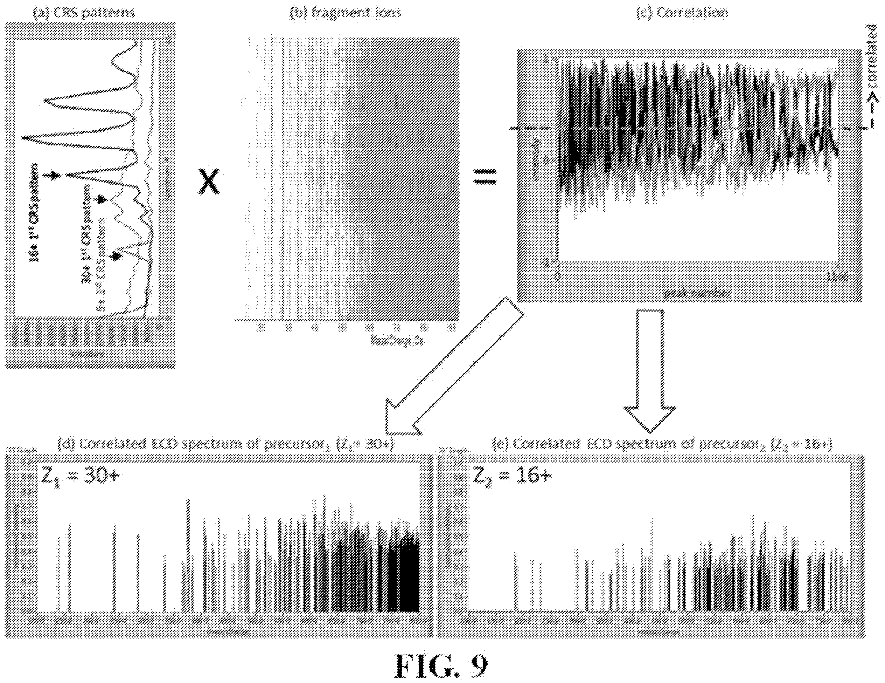

With reference again to FIG. 2, in step 210 the plurality of ExD spectra can be deconvoluted to identify the precursor ion from which detected fragment ions in each ExD spectra were derived from, and thereby resolve the fragment ions corresponding to a particular precursor ion. For example, as illustrated schematically in FIG. 9, the fragment ions in each ExD spectra can be correlated with the charge state pattern determined in step 208 and the fragmentation pattern determined in step 209 to separately identify the m/z of fragment ions resulting from one of the plurality of precursor charge states. In this exemplary embodiment, the correlation ($C30_i$) of FIG. 9c was determined for each fragment peak number i at Z = 30+ depicted in the heat map of FIG. 9b based on the charge state pattern (FIG. 9a) as follows:

$$C30_i = \sum_{m/z} [(I_{30}(m/z) - i_{30}) \times (Frag_i(m/z) - frag_i(m/z))] ,$$

where $I_{30}(m/z)$ represents the normalized 30+ charge state pattern (line in FIG. 9a), where m/z is the scanned precursor m/z, which is the "vertical" scale of FIG. 9b;
where $i_{30}$ is the averaged intensity of the normalized 30+ charge state pattern;
where $Frag_i(m/z)$ is the intensity pattern of normalize ith peak at a specific m/z in FIG. 9b, where m/z is the scanned precursor m/z; and
where $frag_i(m/z)$ is the averaged intensity of normalized ith peak with a specific m/z in FIG. 9b.

It will be appreciated by those skilled in the art that in the above exemplary correlation function, the averaged values are subtracted so as to ease the determination of a threshold for selection. For example, if there is no correlation, the $C30_i$ value should be zero, and if perfectly correlated, the $C30_i$ value should be 1. The threshold can be set at around 0.5, though this or any other threshold can be used in accordance with the present teaching (e.g., 0.3). Similar correlations can likewise be performed for each of the 16+ and 9+ charge states to produce the exemplary deconvoluted spectrum of FIG. 9d (fragment spectrum for precursor Z = 30+ at precursor m/z of 972 Da) and 9e (fragment spectrum for precursor Z = 16+ at precursor m/z of 981 Da), in which fragment ions and the precursor ion from which they are derived can be identified, despite the presence of multiple species of precursor charge states. Further, it should be appreciated that the correlation described above is exemplary only and that other alternative correlation functions or variables can be used in accordance with the present teachings to separate the fragments in ExD spectra corresponding to m/z isolation windows having a plurality of species of precursor ions.

Upon separation of the fragment ions produced from different precursor ions in step 210, the present teachings can also provide for the more definitive characterization of the amino acid sequences of the protein or peptides from which the fragment ions were derived. For example, the presence of carbonic anhydrase in the sample can be confirmed based on the experimentally determined molecular mass of about 29,000 amu for the Z = 30+ precursors (as in step 207), as well as through validation by the deconvoluted spectrum of FIG. 9d, which exhibits m/z c- and z-type fragments that correspond well to the theoretical fragmentation of carbonic anhydrase.

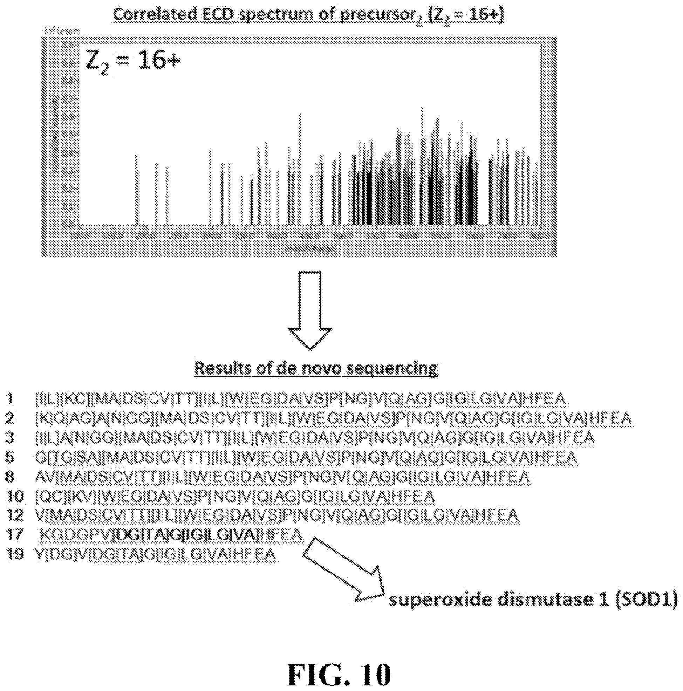

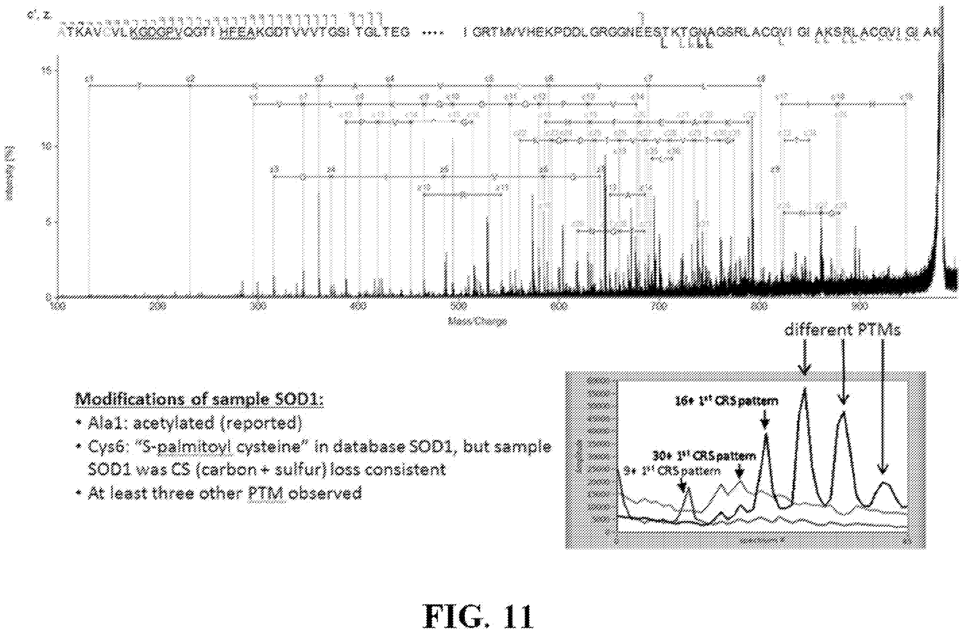

Moreover, while methods and systems in accordance with the present teachings can enable the confirmation and characterization of a known protein in a non-purified sample, the present teachings additionally enable the identification of an unknown protein through the at least partial determination of an amino acid sequence in the protein. By way of example, while the identity of the protein corresponding to the 16+ precursor was initially unknown, the deconvoluted spectrum of FIG. 9e can enable the determination of unique amino acid sequences by *de novo* sequencing within the precursor protein based on the determination of peak pairs, whose separation is matched to the mass of a particular amino acid residue. For example, in step 211, from the correlated ECD spectrum of the precursor having a charge state of 16+ (and the experimentally determined molecular weight of about 15500-16000 amu), various peaks can be compared to identify one or more partial sequences of consecutive amino acids. In step 212, for example, by comparing one or more of the determined sequences depicted in FIG. 10 to a database of known amino acid sequences (e.g., a homology search such as via Protein Prospector available at http://prospector.ucsf.edu/prospector/mshome.htm), a match of sequence 17 positively identifies the protein also present in the sample as superoxide dismutase 1. Thus, methods and systems in accordance with various aspects of the present teachings can not only enable the more definitive characterization of a known protein present in an impure sample, but can also be used in *de novo* sequencing of deconvoluted peptide fragments from an unknown protein, despite the presence of other interfering proteins or peptides. Further, as shown in FIG. 11, after identifying superoxide dismutase 1 as the potential precursor at Z = 16+, the ECD spectrum can again be analyzed to confirm that other amino acid sequences known in superoxide dismutase 1 were also present in the ECD spectrum. Moreover, as indicated in FIG. 11, the changes to the amino acid sequences (e.g., acetylation of Ala1) can further be confirmed based on the partial reconstruction of the detected ECD fragments, as well as a characterization of the various PTM states in the sample based on the existence of small differences in molecular weight of the 16+ superoxide dismutase precursors detected in the various m/z isolation windows.

Those skilled in the art will know or be able to ascertain using no more than routine experimentation, many equivalents to the embodiments and practices described herein. By way of example, the dimensions of the various components and explicit values for particular electrical signals (e.g., amplitude, frequencies, etc.) applied to the various components are merely exemplary and are not intended to limit the scope of the present teachings. Accordingly, it will be understood that the invention is not to be limited to the embodiments disclosed herein, but is to be understood from the following claims, which are to be interpreted as broadly as allowed under the law.

The section headings used herein are for organizational purposes only and are not to be construed as limiting. While the applicant's teachings are described in conjunction with various embodiments, it is not intended that the applicant's teachings be limited to such embodiments. On the contrary, the applicant's teachings encompass various alternatives, modifications, and equivalents, as will be appreciated by those of skill in the art.

**Claims**

1.   A method of processing a sample, comprising:

utilizing an ion source (104) to generate a plurality of precursor peptide or protein ions from a sample solution containing at least one peptide or protein;
scanning a mass range of the precursor ions using a plurality of m/z isolation windows (204);
subjecting the precursor ions within each of the m/z isolation windows to an electron capture or electron transfer dissociation, ExD, reaction (205);

detecting product ions resulting from each of the ExD reactions so as to generate a plurality of ExD spectra corresponding to each of the m/z isolation windows, wherein at least a first ExD spectra corresponding to a first m/z isolation window exhibits one or more ExD fragment ions and one or more charge reduced species of the precursor ions within the first m/z isolation window;

determining a precursor charge state and a molecular weight for one or more species of the precursor ions within the first m/z isolation window at least partially based on a m/z of the said one or more species of precursor ions within the first m/z isolation window and a m/z of the one or more charge reduced species of the precursor ions;

determining a precursor charge state and molecular weight for each of one or more species of precursor ions within each of the m/z isolation windows;

determining a precursor charge state pattern across the mass range for each of the precursor charge states of one or more species of precursor ions exhibiting a similar molecular weight; and

correlating the one or more ExD fragment ions in the ExD spectra corresponding to each respective m/z isolation window with the precursor charge state pattern for each of the one or more species of precursor ions within said respective m/z isolation window.

2. The method of claim 1, further comprising introducing the sample solution to the ion source via direct infusion.

3. The method of claim 1, wherein each of the m/z isolation windows has a range of 1 Da.

4. The method of claim 1, wherein the first ExD spectra corresponding to the first m/z isolation window exhibits a first charge reduced species, and wherein the precursor charge state of a first species of precursor ions ($Z_1$) within the first m/z isolation window is determined as a function of the m/z of the first charged reduced species ($Y_1$) and the m/z of the first species of precursor ions (X), according to the equation:

$$Z_1 = \frac{Y_1}{Y_1 - X};$$

optionally wherein the first ExD spectra further exhibits a second charged reduced species, the method further comprising determining whether the second charge reduced species represents a doubly-reduced species of the first charged reduced species or a charged reduced species of a second species of precursor ions within the first m/z isolation window by the equation:

$$Z_2 = \frac{Y_2}{Y_2 - Y_1};$$

and determining whether $Z_2$ is equal to ($Z_1$-1), where $Y_2$ is the m/z of the second charged reduced species, optionally wherein the molecular weight ($M_1$) of the first species of precursor ions within the first m/z isolation window is determined as a function of the precursor charge state ($Z_1$) and the m/z (X) of the first species of precursor ions according to the equation:

$$M_1 = Z_1 \times (X - m_H),$$

where $m_H$ is the atomic mass of hydrogen.

5. The method of claim 1, wherein the precursor charge state pattern is determined from the relative abundance of each of the one or more species of precursor ions within each of the m/z isolation windows.

6. The method of claim 1, wherein the precursor charge state pattern is determined from the intensity of the singly, charged reduced species of each of the one or more species of precursor ions in the ExD spectra corresponding to each of the m/z isolation windows.

7. The method of claim 1, further comprising generating an ExD spectrum for a first species selected from the one or more species of precursor ions by referring to the precursor charge state pattern, wherein differences in mass between ExD fragment ion peaks in the ExD spectra for the selected first species of the precursor ions are indicative of one or more amino acids in the selected first species of the precursor ions;

said method optionally further comprising at least partially reconstructing the amino acid sequence in the selected first species of the precursor ions;

said method optionally further comprising comparing a partially reconstructed amino acid sequence of the selected first species of the precursor ions to a database of known peptide or protein sequences to identify the peptide or protein ionized to form the first species of the precursor ions.

8. The method of claim 1, further comprising comparing a plurality of the ExD fragment ion spectra corresponding to selected m/z isolation windows, wherein similar ExD fragment ion patterns for a plurality of species of precursor ions of similar molecular weight indicate the presence of different post-translational modifications on similar precursor ions.

9. A mass spectrometer system, comprising:

an ion source (104) configured to generate a plurality of precursor peptide or protein ions from a sample solution containing at least one peptide or protein;

a mass analyzer (106) configured to receive the plurality of precursor ions from the ion source;

an electron capture or electron transfer dissociation, ExD, reaction cell (108) configured to receive the precursor ions transmitted from the mass analyzer and subject the precursor ions to an ExD reaction;

a detector (110); and

a controller (112), wherein the controller is configured to:

scan a mass range of the precursor ions using a plurality of m/z isolation windows to the ExD reaction cell so as to subject the precursor ions within each of the m/z isolation windows to an ExD reaction;

generate a plurality of ExD spectra of product ions resulting from the ExD reactions corresponding to each of the m/z isolation windows, wherein at least a first ExD spectra corresponding to a first m/z isolation window exhibits one or more ExD fragment ions and one or more charge reduced species of the precursor ions within the first m/z isolation window;

determine a precursor charge state and a molecular weight for one or more species of the precursor ions within the first m/z isolation window at least partially based on a m/z of the said one or more species of precursor ions within the first m/z isolation window and a m/z of the one or more charge reduced species of the precursor ions;

wherein the controller is further configured to:

determine a precursor charge state and molecular weight for each of one or more species of precursor ions within each of the m/z isolation windows;

determine a precursor charge state pattern for each of the precursor charge states of one or more species of precursor ions exhibiting a similar molecular weight across the mass range; and

correlate the one or more ExD fragment ions in the ExD spectra corresponding to each respective m/z isolation window with the precursor charge state pattern for each of the one or more species of precursor ions within the respective m/z isolation window.

10. The system of claim 9, further comprising a pump for direct infusion of the sample solution to the ion source.

11. The system of claim 9, wherein each m/z isolation window has a range of 1 Da.

12. The system of claim 9, wherein the first ExD spectra corresponding to the first m/z isolation window exhibits a first charge reduced species,

wherein the precursor charge state ($Z_1$) of a first species of precursor ions within the first m/z isolation window is determined as a function of the m/z of the first charged reduced species ($Y_1$) and the m/z of the first species of precursor ions (X) according to the equation:

$$Z_1 = \frac{Y_1}{Y_1 - X} \, ,$$

and

wherein the molecular weight ($M_1$) of the first species of precursor ions within the first m/z isolation window is determined as a function of the precursor charge state ($Z_1$) and the m/z ($X$) of the first species of precursor ions, according to the equation:

$$M_1 = Z_1 \times (X - m_H),$$

where $m_H$ is the atomic mass of hydrogen.

13. The system of claim 12 wherein the first ExD spectra further exhibits a second charged reduced species, wherein the controller is further configured to determine whether the second charge reduced species represents a doubly-reduced species of the first charged reduced species or a charged reduced species of a second species of precursor ions within the first m/z isolation window by the equation:

$$Z_2 = \frac{Y_2}{Y_2 - Y_1};$$

and determining whether $Z_2$ is equal to (Zi-1), where $Y_2$ is the m/z of the second charged reduced species.

14. The system of claim 9, wherein the controller is further configured to determine the precursor charge state pattern based on the intensity of the singly, charged reduced species of each of the one or more species of precursor ions in the ExD spectra corresponding to each of the m/z isolation windows.

15. The system of claim 9, wherein the controller is further configured to compare a plurality of the ExD fragment ion spectra corresponding to selected m/z isolation windows, wherein similar ExD fragment ion patterns for a plurality of species of precursor ions of similar molecular weight indicate the presence of different post-translational modifications on similar precursor ions.

**Patentansprüche**

1. Verfahren zur Verarbeitung einer Probe, wobei das Verfahren Folgendes umfasst:

Verwenden einer Ionenquelle (104) zum Erzeugen einer Vielzahl von Vorläuferpeptid- oder Proteinionen aus einer Probenlösung, die mindestens ein Peptid oder Protein enthält;
Scannen eines Massenbereichs der Vorläuferionen unter Verwendung einer Vielzahl von m/z-Isolationsfenstern (204);
Unterziehen der Vorläuferionen in jedem der m/z-Isolationsfenster einer Elektroneneinfang- oder Elektronentransferdissoziations-, ExD-, Reaktion (205);
Erfassen von Produkt-Ionen, die aus jeder der ExD-Reaktionen resultieren, um eine Vielzahl von ExD-Spektren zu erzeugen, die jedem der m/z-Isolationsfenster entsprechen; wobei mindestens ein erstes ExD-Spektrum, das einem ersten m/z-Isolationsfenster entspricht, ein oder mehrere ExD-Fragmentionen und eine oder mehrere ladungsreduzierte Spezies der Vorläuferionen innerhalb des ersten m/z-Isolationsfensters aufweist;
Bestimmen eines Vorläuferladungszustands und eines Molekulargewichts für eine oder mehrere Spezies der Vorläuferionen innerhalb des ersten m/z-Isolationsfensters zumindest teilweise basierend auf einem m/z der einen oder mehreren Spezies von Vorläuferionen innerhalb des ersten m/z-Isolationsfensters und einem m/z der einen oder mehreren ladungsreduzierten Spezies der Vorläuferionen;
Bestimmen eines Vorläuferladungszustands und eines Molekulargewichts für jede der einer oder mehreren Spezies von Vorläuferionen innerhalb jedes der m/z-Isolationsfenster;
Bestimmen eines Vorläuferladungszustandsmusters über den Massenbereich für jeden der Vorläuferladungszustände einer oder mehrerer Spezies von Vorläuferionen, die ein ähnliches Molekulargewicht aufweisen; und
Korrelieren des einen oder der mehreren ExD-Fragmentionen in den ExD-Spektren, die jedem jeweiligen m/z-Isolationsfenster entsprechen, mit dem Vorläuferladungszustandsmuster für jede der einen oder mehreren Spezies von Vorläuferionen innerhalb des jeweiligen m/z-Isolationsfensters.

2. Verfahren nach Anspruch 1, das ferner das Einführen der Probenlösung in die Ionenquelle durch direkte Infusion umfasst.

3. Verfahren nach Anspruch 1, wobei jedes der m/z-Isolationsfenster einen Bereich von 1 Da aufweist.

4. Verfahren nach Anspruch 1, wobei die ersten ExD-Spektren, die dem ersten m/z-Isolationsfenster entsprechen, eine erste ladungsreduzierte Spezies aufweisen, und wobei der Vorläuferladungszustand einer ersten Spezies von Vorläuferionen ($Z_1$) innerhalb des ersten m/z-Isolationsfensters als Funktion des m/z der ersten ladungsreduzierten Spezies ($Y_1$) und des m/z der ersten Spezies von Vorläuferionen (X) gemäß folgender Gleichung bestimmt wird:

$$Z_1 = \frac{Y_1}{Y_1 - X};$$

optional wobei die ersten ExD-Spektren ferner eine zweite ladungsreduzierte Spezies zeigen, wobei das Verfahren ferner das Bestimmen umfasst, ob die zweite ladungsreduzierte Spezies eine doppelt reduzierte Spezies der ersten ladungsreduzierten Spezies oder eine ladungsreduzierte Spezies einer zweiten Spezies von Vorläuferionen innerhalb des ersten m/z-Isolationsfensters durch folgende Gleichung darstellt:

$$Z_2 = \frac{Y_2}{Y_2 - Y_1};$$

und Bestimmen, ob $Z_2$ gleich ($Z_1$-1) ist, wobei $Y_2$ das m/z der zweiten ladungsreduzierten Spezies ist, optional wobei das Molekulargewicht ($M_1$) der ersten Spezies von Vorläuferionen innerhalb des ersten m/z-Isolationsfensters als Funktion des Vorläuferladungszustands ($Z_1$) und des m/z (X) der ersten Spezies von Vorläuferionen nach folgender Gleichung bestimmt wird:

$$M_1 = Z_1 \times (X - m_H),$$

wobei $m_H$ die Atommasse von Wasserstoff ist.

5. Verfahren nach Anspruch 1, wobei das Vorläuferladungszustandsmuster aus der relativen Häufigkeit jeder der einen oder mehreren Spezies von Vorläuferionen in jedem der m/z-Isolationsfenster bestimmt wird.

6. Verfahren nach Anspruch 1, wobei das Vorläuferladungszustandsmuster aus der Intensität der einfach ladungsreduzierten Spezies jeder der einen oder mehreren Spezies von Vorläuferionen in den ExD-Spektren bestimmt wird, die jedem der m/z-Isolationsfenster entsprechen.

7. Verfahren nach Anspruch 1, das ferner das Erzeugen eines ExD-Spektrums für eine erste Spezies, ausgewählt aus der einen oder den mehreren Spezies von Vorläuferionen, unter Bezugnahme auf das Vorläuferladungszustandsmuster umfasst, wobei Massenunterschiede zwischen ExD-Fragmentionenspitzenwerten in den ExD-Spektren für die ausgewählte erste Spezies der Vorläuferionen eine oder mehrere Aminosäuren in der ausgewählten ersten Spezies der Vorläuferionen anzeigen; wobei das Verfahren optional ferner das zumindest teilweise Rekonstruieren der Aminosäuresequenz in der ausgewählten ersten Spezies der Vorläuferionen umfasst; wobei das Verfahren optional ferner das Vergleichen einer teilweise rekonstruierten Aminosäuresequenz der ausgewählten ersten Spezies der Vorläuferionen mit einer Datenbank bekannter Peptid- oder Proteinsequenzen umfasst, um das Peptid oder Protein zu identifizieren, das ionisiert wurde, um die erste Spezies der Vorläuferionen zu bilden.

8. Verfahren nach Anspruch 1, das ferner das Vergleichen einer Vielzahl der ExD-Fragmentionenspektren umfasst, die ausgewählten m/z-Isolationsfenstern entsprechen, wobei ähnliche ExD-Fragmentionenmuster für eine Vielzahl von Spezies von Vorläuferionen mit ähnlichem Molekulargewicht das Vorhandensein verschiedener posttranslationaler Modifikationen an ähnlichen Vorläuferionen anzeigen.

9. Massenspektrometersystem, das Folgendes umfasst:

eine Ionenquelle (104), die dafür konfiguriert ist, eine Vielzahl von Vorläuferpeptid- oder Proteinionen aus einer Probenlösung zu erzeugen, die mindestens ein Peptid oder Protein enthält;
einen Massenanalysator (106), der dafür konfiguriert ist, die Vielzahl von Vorläuferionen von der Ionenquelle zu empfangen;
eine Elektroneneinfang- oder Elektronentransferdissoziations-ExD-Reaktionszelle (108), die dafür konfiguriert ist, die vom Massenanalysator übertragenen Vorläuferionen aufzunehmen und die Vorläuferionen einer ExD-Reaktion zu unterziehen;
einen Detektor (110); und
eine Steuerung (112), wobei die Steuerung für Folgendes konfiguriert ist:

Scannen eines Massenbereichs der Vorläuferionen unter Verwendung einer Vielzahl von m/z-Isolationsfenstern zur ExD-Reaktionszelle, um die Vorläuferionen in jedem der m/z-Isolationsfenster einer ExD-Reaktion zu unterziehen;
Erzeugen einer Vielzahl von EXD-Spektren von Produkt-Ionen, die aus den ExD-Reaktionen resultieren, die jedem der m/z-Isolationsfenster entsprechen, wobei mindestens ein erstes ExD-Spektrum, das einem ersten m/z-Isolationsfenster entspricht, ein oder mehrere ExD-Fragmentionen und eine oder mehrere ladungsreduzierte Spezies der Vorläuferionen innerhalb des ersten m/z-Isolationsfensters aufweist;
Bestimmen eines Vorläuferladungszustands und eines Molekulargewichts für eine oder mehrere Spezies der Vorläuferionen innerhalb des ersten m/z-Isolationsfensters zumindest teilweise basierend auf einem m/z der einen oder mehreren Spezies von Vorläuferionen innerhalb des ersten m/z-Isolationsfensters und einem m/z der einen oder mehreren ladungsreduzierten Spezies der Vorläuferionen;

wobei die Steuerung ferner für Folgendes konfiguriert ist:

Bestimmen eines Vorläuferladungszustands und eines Molekulargewichts für jede der einer oder mehreren Spezies von Vorläuferionen innerhalb jedes der m/z-Isolationsfenster;
Bestimmen eines Vorläuferladungszustandsmusters für jeden der Vorläuferladungszustände einer oder mehrerer Spezies von Vorläuferionen, die ein ähnliches Molekulargewicht aufweisen, über den Massenbereich; und
Korrelieren des einen oder der mehreren ExD-Fragmentionen in den ExD-Spektren, die dem jeweiligen m/z-Isolationsfenster entsprechen, mit dem Ladungszustandsmuster des Vorläufers für jede der einen oder mehreren Spezies von Vorläuferionen innerhalb des jeweiligen m/z-Isolationsfensters.

10. System nach Anspruch 9, das ferner eine Pumpe zur direkten Infusion der Probenlösung zur Ionenquelle umfasst.

11. System nach Anspruch 9, wobei jedes m/z-Isolationsfenster einen Bereich von 1 Da aufweist.

12. System nach Anspruch 9, wobei die ersten ExD-Spektren, die dem ersten m/z-Isolationsfenster entsprechen, eine erste ladungsreduzierte Spezies aufweisen,
wobei der Vorläuferladungszustand ($Z_1$) einer ersten Spezies von Vorläuferionen innerhalb des ersten m/z-Isolationsfensters als Funktion des m/z der ersten ladungsreduzierten Spezies ($Y_1$) und des m/z der ersten Spezies von Vorläuferionen (X) gemäß folgender Gleichung bestimmt wird:

$$Z_1 = \frac{Y_1}{Y_1 - X}$$

und
wobei das Molekulargewicht ($M_1$) der ersten Spezies von Vorläuferionen innerhalb des ersten m/z-Isolationsfensters als Funktion des Vorläuferladungszustands ($Z_1$) und des m/z (X) der ersten Spezies von Vorläuferionen nach folgender Gleichung bestimmt wird:

$$M_1 = Z_1 \times (X - m_H),$$

wobei $m_H$ die Atommasse von Wasserstoff ist.

13. System nach Anspruch 12, wobei die ersten ExD-Spektren ferner eine zweite ladungsreduzierte Spezies zeigen, wobei die Steuerung ferner dafür konfiguriert ist, durch folgende Gleichung zu bestimmen, ob die zweite ladungsreduzierte Spezies eine doppelt reduzierte Spezies der ersten ladungsreduzierten Spezies oder eine ladungsreduzierte Spezies einer zweiten Spezies von Vorläuferionen innerhalb des ersten m/z-Isolationsfensters darstellt:

$$Z_2 = \frac{Y_2}{Y_2 - Y_1};$$

und Bestimmen, ob $Z_2$ gleich ($Z_1$-1) ist, wobei $Y_2$ das m/z der zweiten ladungsreduzierten Spezies ist.

14. System nach Anspruch 9, wobei die Steuerung ferner dafür konfiguriert ist, das Vorläuferladungszustandsmuster basierend auf der Intensität der einfach ladungsreduzierten Spezies jeder der einen oder mehreren Spezies von Vorläuferionen in den ExD-Spektren zu bestimmen, die jeweils den m/z-Isolationsfenstern entsprechen.

15. System nach Anspruch 9, wobei die Steuerung ferner dafür konfiguriert ist, eine Vielzahl der ExD-Fragmentionenspektren zu vergleichen, die ausgewählten m/z-Isolationsfenstern entsprechen, wobei ähnliche ExD-Fragmentionenmuster für eine Vielzahl von Spezies von Vorläuferionen mit ähnlichem Molekulargewicht das Vorhandensein verschiedener posttranslationaler Modifikationen an ähnlichen Vorläuferionen anzeigen.

**Revendications**

1. Procédé de traitement d'un échantillon, comprenant :

l'utilisation d'une source d'ions (104) pour générer une pluralité d'ions peptidiques ou protéiniques précurseurs à partir d'une solution d'échantillon contenant au moins un peptide ou une protéine ;
le balayage d'un gamme de masse des ions précurseurs en utilisant une pluralité de fenêtres d'isolation m/z (204) ;
la soumission des ions précurseurs à l'intérieur de chacune des fenêtres d'isolation m/z à une réaction de dissociations de capture d'électrons ou de transfert d'électrons, ExD, (205) ;
la détection d'ions-produits résultant de chacune des réaction ExD afin de générer une pluralité de spectres ExD correspondant à chacune des fenêtres d'isolation m/z, dans lequel au moins un premier spectre ExD correspondant à une premier fenêtre d'isolation m/z présente un ou plusieurs ions-fragments ExD et une ou plusieurs espèces à charges réduites des ions précurseurs à l'intérieur de la première fenêtre d'isolation m/z ;
la détermination d'un état de charge de précurseur et d'un poids moléculaire pour une ou plusieurs espèces des ions précurseurs à l'intérieur de la première fenêtre d'isolation m/z au moins partiellement sur la base d'un m/z desdites une ou plusieurs espèces d'ions précurseurs à l'intérieur de la première fenêtre d'isolation m/z et d'un m/z des une ou plusieurs espèces à charges réduites des ions précurseurs ;
la détermination d'un état de charge de précurseur et d'un poids moléculaire pour chacune d'une ou de plusieurs espèces d'ions précurseurs à l'intérieur de chacune des fenêtres d'isolation m/z ;
la détermination d'un type d'état de charge de précurseur sur la gamme de masse pour chacun des états de charge de précurseur d'une ou de plusieurs espèces d'ions précurseurs présentant un poids moléculaire similaire ; et
la corrélation des un ou plusieurs ions-fragments ExD dans les spectres ExD correspondant à chaque fenêtre d'isolation m/z respective avec le type d'état de charge de précurseur pour chacune des un ou plusieurs espèces d'ions précurseurs à l'intérieur de ladite fenêtre d'isolation m/z respective.

2. Procédé selon la revendication 1, comprenant en outre l'introduction de la solution d'échantillon dans la source d'ions par l'intermédiaire d'infusion directe.

**3.** Procédé selon la revendication 1, dans lequel chacune des fenêtres d'isolation m/z a une gamme d' 1 Da.

**4.** Procédé selon la revendication 1, dans lequel le premier spectres ExD correspondant à la première fenêtre d'isolation m/z présente une première espèce à charge réduite, et dans lequel l'état de charge de précurseur d'une première espèce d'ions précurseurs ($Z_1$) à l'intérieur de la première fenêtre d'isolation m/z est déterminé en fonction du m/z de la première espèce à charge réduite ($Y_1$) et du m/z de la première espèce d'ions précurseurs (X), selon l'équation :

$$Z_1 = \frac{Y_1}{Y_1 - X};$$

optionnellement dans lequel le premier spectre ExD présente en outre une seconde espèce à charge réduite, le procédé comprenant en outre la détermination du fait que la seconde espèce à charge réduite représente une espèce doublement réduite de la première espèce à charge réduite ou une espèce à charge réduite d'une seconde espèce d'ions précurseurs à l'intérieur de la première fenêtre d'isolation m/z par l'équation :

$$Z_2 = \frac{Y_2}{Y_2 - Y_1};$$

et la détermination du fait que $Z_2$ est ou non égal à ($Z_1$-l), où $Y_2$ est le m/z de la seconde espèce à charge réduite, optionnellement dans lequel le poids moléculaire ($M_1$) de la première espèce d'ions précurseurs à l'intérieur de la première fenêtre d'isolation m/z est déterminé en fonction de l'état de charge de précurseur ($Z_1$) et du m/z (X) de la première espèce d'ions précurseurs selon l'équation :

$$M_1 = Z_1 \times (X - m_H),$$

où $m_H$ est la masse atomique d'hydrogène.

**5.** Procédé selon la revendication 1, dans lequel le type d'état de charge de précurseur est déterminé à partir de l'abondance relative de chacune des une ou plusieurs espèces d'ions précurseurs à l'intérieur de chacune des fenêtres d'isolation m/z.

**6.** Procédé selon la revendication 1, dans lequel le type d'état de charge de précurseur est déterminé à partir de l'intensité de l'espèce à charge individuellement réduite de chacune des une ou plusieurs espèces d'ions précurseurs dans les spectres ExD correspondant à chacune des fenêtres d'isolation m/z.

**7.** Procédé selon la revendication 1, comprenant en outre la génération d'un spectre ExD pour une première espèce sélectionnée à partir des une ou plusieurs espèces d'ions précurseurs en faisant référence au type d'état de charge de précurseur, dans lequel des différences de masse entre des pics d'ions-fragments ExD dans les spectres ExD pour la première espèce sélectionnée des ions précurseurs sont indicatives d'un ou de plusieurs acides aminés dans la première espèce sélectionnée des ions précurseurs ;
ledit procédé comprenant optionnellement en outre la reconstruction au moins partielle de la séquence d'acides aminés dans la première espèce sélectionnée des ions précurseurs ;
ledit procédé comprenant optionnellement en outre la comparaison d'une séquence d'acides aminés partiellement reconstruite de la première espèce sélectionnée des ions précurseurs à une base de données de séquences peptidiques ou protéiniques connues pour identifier le peptide ou la protéine ionisé pour former la première espèce des ions précurseurs.

**8.** Procédé selon la revendication 1, comprenant en outre la comparaison d'une pluralité des spectres d'ions-fragments ExD correspondant à des fenêtres d'isolation m/z sélectionnées, dans lequel des types d'ions-fragments ExD similaires pour une pluralité d'espèces d'ions précurseurs de poids moléculaire similaire indiquent la présence de différentes modifications post-translationnelles sur des ions précurseurs similaires.

9. Système spectromètre de masse, comprenant :

une source d'ions (104) configurée pour générer une pluralité d'ions peptidiques ou protéiniques précurseurs à partir d'une solution d'échantillon contenant au moins un peptide ou une protéine ;
un analyseur de masse (106) configuré pour recevoir la pluralité d'ions précurseurs à partir de la source d'ions ;
une cellule de réaction de dissociations de capture d'électrons ou de transfert d'électrons, ExD, (108) configurée pour recevoir les ions précurseurs transmis à partir de l'analyseur de masse et soumettre les ions précurseurs à une réaction ExD ;
un détecteur (110) ; et
une unité de commande (112), dans lequel l'unité de commande est configurée pour :

balayer une gamme de masse des ions précurseurs en utilisant une pluralité de fenêtres d'isolation m/z sur la cellule de réaction ExD afin de soumettre les ions précurseurs à l'intérieur de chacune des fenêtres d'isolation m/z à une réaction ExD ;
générer une pluralité de spectres ExD d'ions-produits résultant des réaction ExD correspondant à chacune des fenêtres d'isolation m/z, dans lequel au moins un premier spectre ExD correspondant à une première fenêtre d'isolation m/z présente un ou plusieurs ions-fragments ExD et une ou plusieurs espèces à charges réduites des ions précurseurs à l'intérieur de la première fenêtre d'isolation m/z ;
déterminer un état de charge de précurseur et un poids moléculaire pour une ou plusieurs espèces des ions précurseurs à l'intérieur de la première fenêtre d'isolation m/z au moins partiellement sur la base d'un m/z desdites une ou plusieurs espèces d'ions précurseurs à l'intérieur de la première fenêtre d'isolation m/z et d'un m/z des une ou plusieurs espèces à charges réduites des ions précurseurs ;

dans lequel l'unité de commande est en outre configurée pour :

déterminer un état de charge de précurseur et un poids moléculaire pour chacune d'une ou de plusieurs espèces d'ions précurseurs à l'intérieur de chacune des fenêtres d'isolation m/z ;
déterminer un type d'état de charge de précurseur pour chacun des états de charge de précurseur d'une ou de plusieurs espèces d'ions précurseurs présentant un poids moléculaire similaire sur la gamme de masse ; et
corréler les un ou plusieurs ions-fragments ExD dans les spectres ExD correspondant à chaque fenêtre d'isolation m/z respective avec le type d'état de charge de précurseur pour chacune des une ou plusieurs espèces d'ions précurseurs à l'intérieur de la fenêtre d'isolation m/z respective.

10. Système selon la revendication 9, comprenant en outre une pompe pour infusion directe de la solution d'échantillon dans la source d'ions.

11. Système selon la revendication 9, dans lequel chaque fenêtre d'isolation m/z a une gamme d' 1 Da.

12. Système selon la revendication 9, dans lequel le premier spectre ExD correspondant à la première fenêtre d'isolation m/z présente une première espèce à charge réduite,
dans lequel l'état de charge de précurseur ($Z_1$) d'une première espèce d'ions précurseurs à l'intérieur de la première fenêtre d'isolation m/z est déterminé en fonction du m/z de la première espèce à charge réduite ($Y_1$) et du m/z de la première espèce d'ions précurseurs (X) selon l'équation :

$$Z_1 = \frac{Y_1}{Y_1 - X} ,$$

et
dans lequel le poids moléculaire ($M_1$) de la première espèce d'ions précurseurs à l'intérieur de la première fenêtre d'isolation m/z est déterminé en fonction de l'état de charge de précurseur ($Z_1$) et du m/z (X) de la première espèce d'ions précurseurs, selon l'équation :

$$M_1 = Z_1 \times (X - m_H),$$

où $m_H$ est la masse atomique d'hydrogène.

13. Système selon la revendication 12 dans lequel le premier spectre ExD présente en outre une seconde espèce à charge réduite, dans lequel l'unité de commande est en outre configurée pour déterminer le fait que la seconde espèce à charge réduite représente une espèce doublement réduite de la première espèce à charge réduite ou une espèce à charge réduite d'une seconde espèce d'ions précurseurs à l'intérieur de la première fenêtre d'isolation m/z par l'équation :

$$Z_2 = \frac{Y_2}{Y_2 - Y_1};$$

et la détermination du fait que $Z_2$ est ou non égal à ($Z_1$-1), où Y est le m/z de la seconde espèce à charge réduite.

14. Système selon la revendication 9, dans lequel l'unité de commande est en outre configurée pour déterminer le type d'état de charge de précurseur sur la base de l'intensité de l'espèce à charge individuellement réduite de chacune des un ou plusieurs espèces d'ions précurseurs dans les spectres ExD correspondant à chacune des fenêtres d'isolation m/z.

15. Système selon la revendication 9, dans lequel l'unité de commande est en outre configurée pour comparer une pluralité des spectres d'ions-fragments ExD correspondants à des fenêtres d'isolation m/z sélectionnées, dans lequel des types d'ions-fragments ExD similaires pour une pluralité d'espèces d'ions précurseurs de poids moléculaire similaire indiquent la présence de différentes modifications post-translationnelles sur des ions précurseurs similaires.

**FIG. 1**

201 — Deliver Sample to Ion Source

202 — Ionize Sample

203 — Obtain MS Survey Scan

204 — Precursor Scan On Narrow m/z Isolation Windows

205 — Perform ExD Reaction On Each m/z Window

206 — Obtain ExD Spectra for Each m/z Window

207 — Determine Precursor Charge State and MW

208 — Determine Charge State Pattern

209 — Determine Fragmentation Pattern For Charge States

210 — Deconvolute Plurality of ExD Spectra

211 — Sequence Amino Acids Of Correlated ExD Spectra

212 — Identify Protein Based on Amino Acids

200

**FIG. 2**

**FIG. 3**

FIG. 4

FIG. 5

**FIG. 6**

**FIG. 7**

FIG. 8

(a) CRS patterns

(b) fragment ions

(c) Correlation

(d) Correlated ECD spectrum of precursor₁ (Z₁ = 30+)
Z₁ = 30+

(e) Correlated ECD spectrum of precursor₂ (Z₂ = 16+)
Z₂ = 16+

FIG. 9

Correlated ECD spectrum of precursor$_2$ (Z$_2$ = 16+)

$Z_2 = 16+$

Results of de novo sequencing

1 [I|L][KC][MA|DS|CV|TT][I|L][W|EG|DA|VS]P[NG]V[Q|AG]G[I|G|LG|VA]HFEA
2 [K|Q|AG|A|N|GG][MA|DS|CV|TT][I|L][W|EG|DA|VS]P[NG]V[Q|AG]G[I|G|LG|VA]HFEA
3 [I|L|A|N|GG][MA|DS|CV|TT][I|L][W|EG|DA|VS]P[NG]V[Q|AG]G[I|G|LG|VA]HFEA
5 G[TG|SA][MA|DS|CV|TT][I|L][W|EG|DA|VS]P[NG]V[Q|AG]G[I|G|LG|VA]HFEA
8 AV[MA|DS|CV|TT][I|L][W|EG|DA|VS]P[NG]V[Q|AG]G[I|G|LG|VA]HFEA
10 [QC][KV][W|EG|DA|VS]P[NG]V[Q|AG]G[I|G|LG|VA]HFEA
12 V[MA|DS|CV|TT][I|L][W|EG|DA|VS]P[NG]V[Q|AG]G[I|G|LG|VA]HFEA
17 KGDGPV[DG|TA]G[I|G|LG|VA]HFEA
19 Y[DG]V[DG|TA]G[I|G|LG|VA]HFEA

superoxide dismutase 1 (SOD1)

FIG. 10

Modifications of sample SOD1:
* Ala1: acetylated (reported)
* Cys6: "S-palmitoyl cysteine" in database SOD1, but sample SOD1 was CS (carbon + sulfur) loss consistent
* At least three other PTM observed

**FIG. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6995366 B
- US 7145139 B
- US 7775034 B
- US 7714283 B
- WO 2014191821 A

**Non-patent literature cited in the description**

- **JAMES W. HAGER ; J. C. YVES LE BLANC.** Product ion scanning using a Q-q-Qlinear ion trap (Q TRAP®) mass spectrometer. *Rapid Communications in Mass Spectrometry,* 2003, vol. 17, 1056-1064
- **BABA et al.** Electron Capture Dissociation in a Radio Frequency Ion Trap. *Anal. Chem.,* 01 August 2004, vol. 76 (15), 4263-6